# EUROPEAN PATENT SPECIFICATION

(11) **EP 0 026 289 B1**
(45) Date of publication and mention of the grant of the patent: **02.06.1982**
(21) Application number: 80104365.4
(22) Date of filing: 24.07.1980
(51) Int. Cl.: C07C 59/56, C07C 59/68, C07C 69/612, C07C 69/732, C07C 69/734, C07C 79/46, C07C 103/34, A61K 31/04, A61K 31/16, A61K 31/19

(54) **Antihypertensive polyhalohydroxyisopropyl phenylalkanoic and phenylalkenoic acids, amides and esters, pharmaceutical compositions therefrom and intermediates thereto**
Antihypertensive Polyhalohydroxyisopropylphenylalkan- und -alkensäuren, deren Amide und Ester, pharmazeutische Mischungen daraus und Zwischenprodukte hierzu
Acides polyhalohydroxyisopropylphénylalcane et -alcène antihypertensifs, amides et esters, compositions pharmaceutiques en résultant et leurs produits intermédiaires

(30) Priority: 26.07.1979 US 61045
(43) Date of publication of application: 08.04.1981
(73) Proprietor: E.I. DU PONT DE NEMOURS AND COMPANY, Wilmington Delaware 19898 (US)
(72) Inventor: Aldrich, Paul Edward, Sharpley Wilmington Delaware 19803 (US); Berezin, Gilbert Harvey, Huntington New York 11743 (US)
(74) Representative: von Kreisler, Alek, Dipl.-Chem.

## Description

### Background of the Invention

This invention relates to acids and derivatives thereof having useful pharmaceutical properties. In particular, this invention relates to phenylalkanoic and phenylalkenoic acids and derivatives thereof which are useful as antihypertensive agents.

U.S. Patent 4,058,612 issued to Neustadt on November 15, 1977 discloses 6-(polyhaloisopropyl)quinazoline-2,4-diones which are useful agents in treatment of mammalian hypertension. The compounds are prepared by reacting 2-(lower alkoxycarbonyl)aniline with a polyhalo acetone or hydrate thereof, followed by reaction of the resulting product with the appropriate isocyanate to form a urea, and hydrolysis of the urea.

U.S. Patent 4,103,018 issued to Neustadt et al. on July 25, 1978 discloses 2-[4-(polyhalo-2-hydroxy-2-propyl)anilino]-2-oxazolin-4-ones and thiazolin-4-ones corresponding thereto, which have useful antihypertensive properties. The compounds are prepared by reaction of the appropriate 4-(polyhalo-2-hydroxy-2-propyl)aniline with a β-chloroethanoyl isocyanate or isothiocyanate and then cyclization of the resultant intermediate.

Many current antihypertensive agents produce unwanted side effects because of undesirable mechanisms of action. For example, mecamylamine is a ganglion blocker; phenoxybenzamide is an α-adrenergic receptor blocker; and reserpine is a cetecholamine depletor. Each of these mechanism of action is undesirable because serious side-effects are produced. There is a constant need for antihypertensive agents which do not produce the side effects, which have fewer side effects, or which minimize such adverse side effects.

### Summary of the Invention

According to the present invention there are provided compounds of formula I, processes for their manufacture, and pharmaceutical compositions containing them. 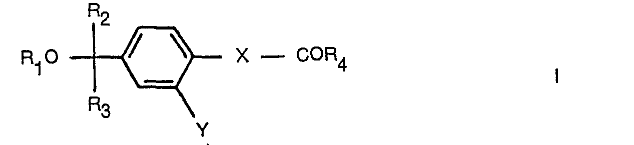 wherein
R, is hydrogen, alkyl of 1-6 carbon atoms, or acyl of 1-6 carbon atoms;
Y is hydrogen, NO₂, alkoxy of 1-3 carbon atoms, or alkylthio of 1-3 carbon atoms; 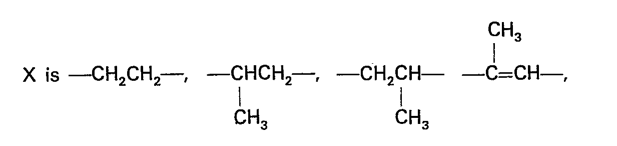 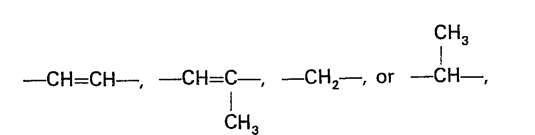 with the proviso that when Y is H, X is other than - 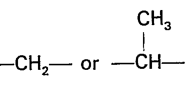 and when Y is alkylthio, X is other than - 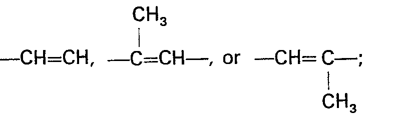
R₄ is N(CH₃)₂, O-Z, S-Z, or CI provided that when Y is H and X is 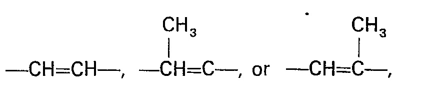 R₄ is other than N(CH₃)₂, wherein
Z is H, alkyl of 1-10 carbon atoms, alkenyl of 3-10 carbon atoms, aryl or aralkyl in which the alkyl group has 1-6 carbon atoms,
with the proviso that when Z is aryl or aralkyl, the aryl group has the formula 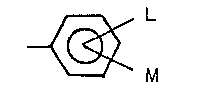 wherein L and M are independently selected from the group consisting of H, F, Cl, Br, NO₂, alkyl of 1-4 carbon atoms, alkoxy of 1-4 carbon atoms, CF₃, OCF₃, CN, phenyl, and COOR' where
R' is H, CH₃ or C₂H₅; and
R₂ and R₃ are independently CF₃, CF_{z}CI, or CF₂H.

Compounds where R₄ is CI are useful solely as intermediates to the antihypertensive agents of the invention.

### Detailed Description of the Invention

The compounds of the invention, excluding those wherein R₄ is Cl, are useful as antihypertensive agents. Compounds wherein R₄ is CI are useful as intermediates to the antihypertensive agents of the invention. The compounds of this invention are believed to lower blood pressure by a desirable mechanism of action, i.e., direct peripheral vasodilation and, therefore have a distinct advantage over antihypertensive agents having undesirable mechanisms of action. Moreover, the compounds of the invention have been shown not to produce central nervous system effects such as those encountered in the use of clonidine and a-methyldopa.

### Preferred Compounds

Compounds of formula I which are preferred for their antihypertensive activity are those wherein, independently,
Rᵢ is hydrogen;
Y is OCH₃ or OC₂H₅;
X is ―CH₂CH₂―;
R₄ is OH, OCH₃, OC₂H₅, or N(CH₃)₂; or
R₂ and R₃ are each CF₃;
More preferred compounds are compounds of formula I in which R₁ is hydrogen;
Y is OCH₃ or OC₂H₅;
X is ―CH₂CH₂―;
R₄ is OH, OCH₃, OC₂H₅, or N(CH₃)₂; and
R₂ and R₃ are CF₃.

Specifically or most preferred are the following compounds where Y is OCH₃:
(a) 3-{2-methoxy-4-[2,2,2-trifluoro-1-hydroxy-1-(trifluoromethyl)ethyl]phenyl}propanoic acid;
(b) methyl 2-methoxy-4-[2,2,2-trifluoro-1-hydroxy-1-(trifluoromethyl)ethyl]benzenepropanoate;
(c) ethyl 2-methoxy-4-[2,2,2-trifluoro-1-hydroxy-1-(trifluoromethyl)ethyl]benzenepropanoate; and
(d) 3-{2-methoxy-4-[2,2,2-trifluoro-1-hydroxy-1-(trifluoromethyl)ethyl]phenyl}-N,N-dimethylpro- panamide.

### Synthesis

Compounds of formula I wherein Y is H, NO₂, or alkylthio of 1-3 carbon atoms and X is 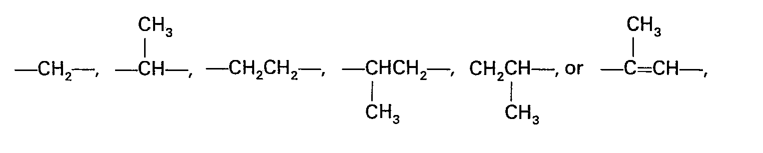 are prepared by a process which comprises first contacting a compound of the formula 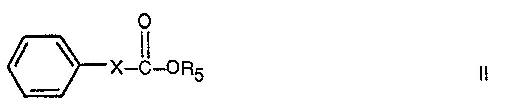 wherein
R₅ is an alkyl group of 1-4 carbon atoms and
X is other than 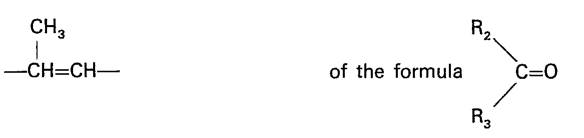 where R₂ and R₃ are as previously defined in the presence of a suitable catalyst under a pressure near autogenous pressure or above and at a temperature of from about 10° to 150°C, preferably from about 25° to 50°C, for from about 4-16 hours. Suitable catalysts include Friedel-Crafts catalysts, such as AICl, or BF₃. This reaction is represented by the following equation: 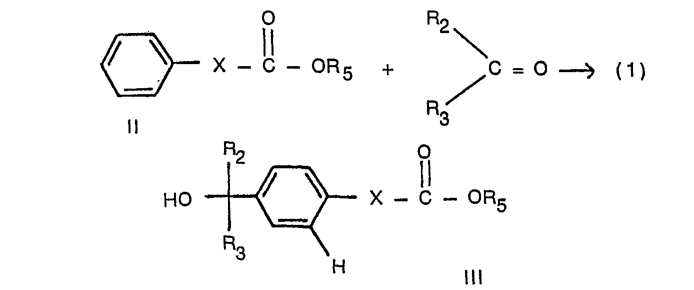 Hydrolysis of a compound of formula III with a suitable base, such as sodium hydroxide, in water or other suitable solvent gives the corresponding phenylalkanoic acid.

Compounds of formula I wherein X is- 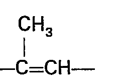 are prepared pursuant to equation (2). 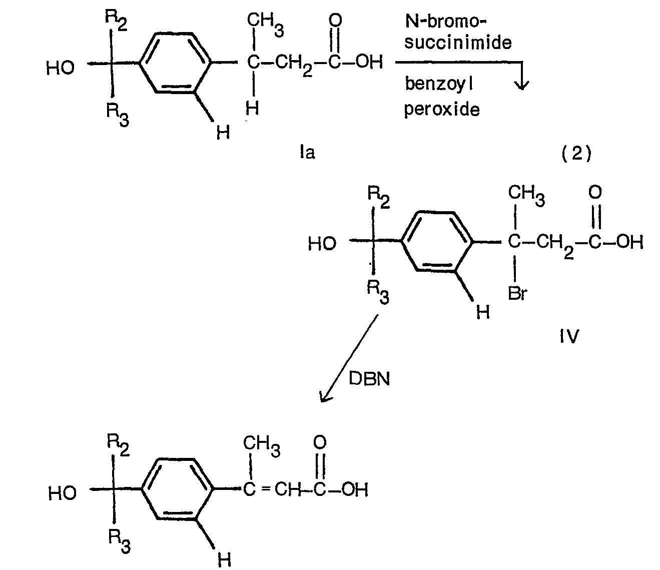 A phenylalkanoic acid prepared by hydrolysis of the product of equation (1) is contacted with a suitable brominating agent, such as N-bromosuccinimide, in a suitable solvent, such as carbon tetrachloride, in the presence of a suitable catalyst, such as benzoyl peroxide, to obtain a compound of formula IV which is then reacted with a suitable dehydrohalogenation agent, such as 1,5-diazabicyclo[3.4.0]nonene-5(DBN), to give the desired compound of the invention.

Compounds of formula I wherein Y is N0₂ are prepared by contacting an acid of formula I with a nitrating agent such as fuming nitric acid in the presence of concentrated sulfuric acid, preferably at 10°-15°C.

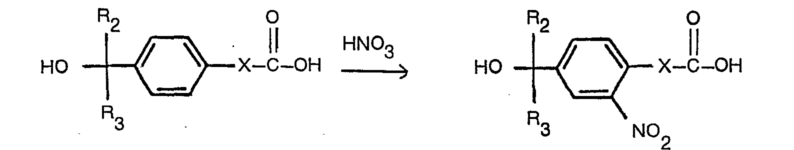

Compounds of formula I wherein Y is an alkylthio group of 1-3 carbon atoms are prepared pursuant to equation (3). 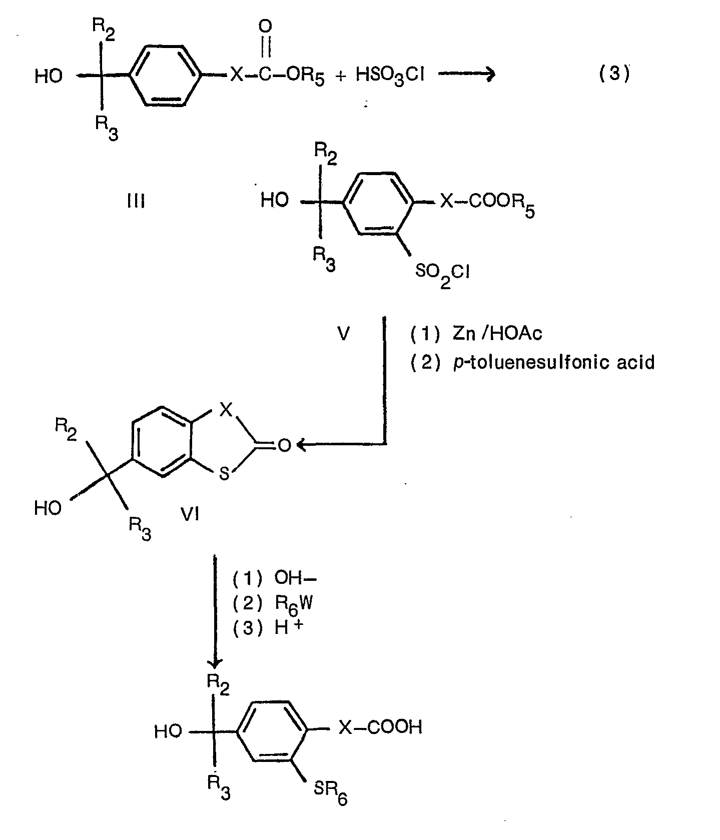 where R₆ is alkyl of 1-3 carbon atoms. A compound of formula III is contacted with excess chlorosulfonic acid to obtain a sulfonyl chloride derivative, V. Compound V is reduced with zinc in acetic acid (HOAc) to obtain a reaction product which is then treated with p-toluenesulfonic acid in toluene to obtain a compound of formula VI. The compound of formula VI is contacted with a suitable base, such as sodium hydroxide, in a suitable solvent, such as dimethylacetamide and ethanol, to obtain a reaction product which is then treated with an alkyl halide, R₆W where R₆ is an alkyl group of from 1 to 3 carbon atoms and W is chloride, bromide or iodide, to obtain a reaction product that after acidification gives a compound of the invention.

Compounds of formula I wherein Y is H, X is - 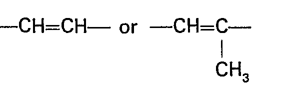 are prepared by another process of the invention. Toluene is contacted with a polyhaloketone of the formula 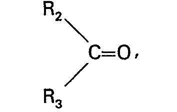 wherein R₂ and R₃ are as previously defined, in the presence of a suitable Friedel-Crafts catalyst, such as AICI₃, under a pressure at least equivalent to autogenous pressure of the polyhaloketone at a temperature of from about 10° to 150°C, preferably from 25° to 50°C, to obtain a compound of formula VII. The reaction is conveniently performed by utilizing a sealed reactor. The compound of VII is oxidized with Cr0₃ in acetic anhydride to obtain a compound of formula VIII which is then contacted with malonic acid or methylmalonic acid, preferably in a pyridine-piperidine solution, to obtain a compound of the invention. The process can be represented as follows: 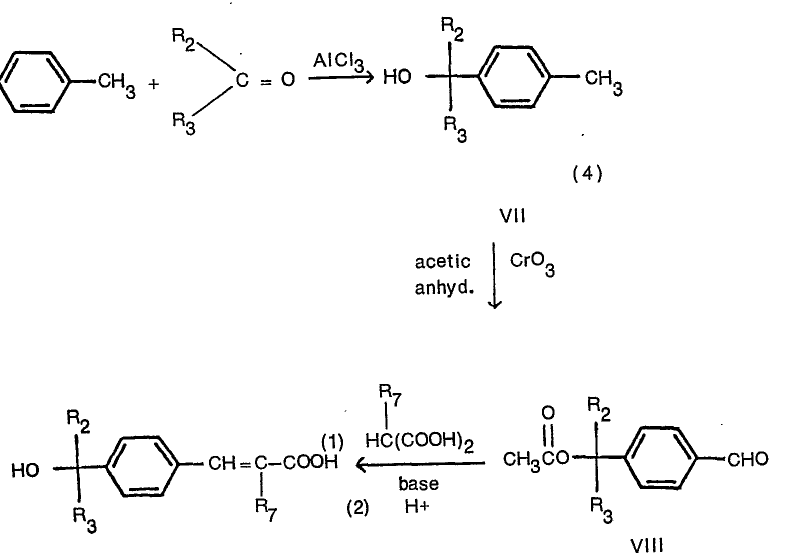 wherein R₇=H or CH₃.

Compounds of formula I wherein R, is H and Y is alkoxy of 1-3 carbon atoms are prepared by the process represented by equation (5), wherein R₆ and R₇ are as previously defined.

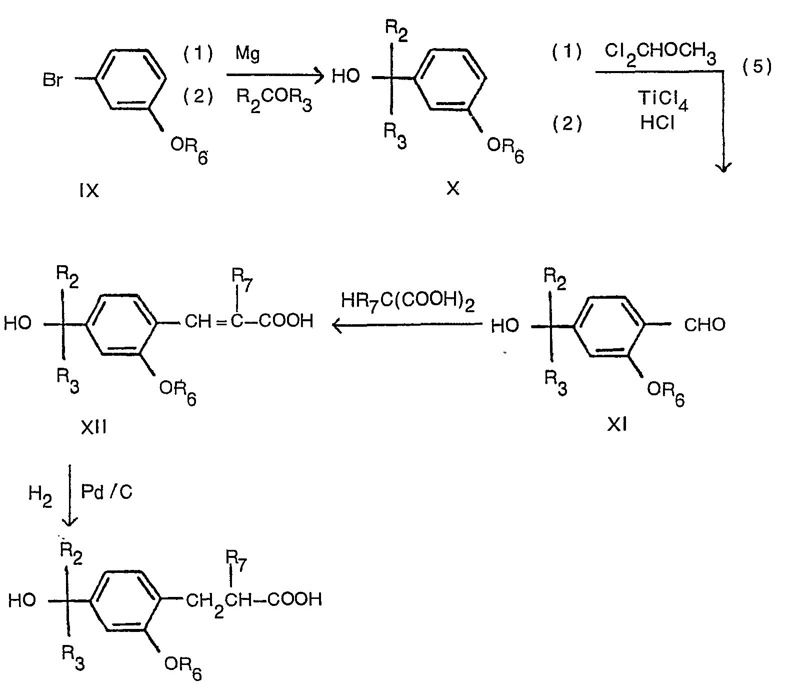

A meta-bromoalkoxybenzene or meta-chloroalkoxybenzene of formula IX is treated with magnesium in a solvent such as tetrahydrofuran at a temperature up to the boiling point of the solvent to produce a magnesium halide derivative to which is then added a polyhaloketone to give a compound of formula X. The compound of formula X is contacted with a,a-dichloromethyl methyl ether in a suitable solvent, such as dichloromethane, in the presence of a suitable catalyst, such as titanium tetrachloride, followed by treatment with aqueous HCI to obtain a reaction product represented by formula XI. This reaction product is treated with malonic acid or methylmalonic acid in a solvent such as pyridine containing a base such as piperidine to obtain an acid of formula I wherein X is 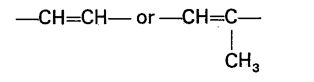 which can be reduced with hydrogen in the presence of a suitable catalyst, such as 10% palladium on carbon, to produce an acid of formula I wherein X is

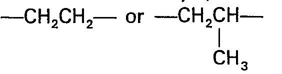

Compounds of formula I having X equal to 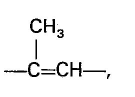 Y is alkoxy of 1-3 carbon atoms, and R₄ is OCH₃ are prepared by a process represented by equation (6) wherein R₆ is as previously defined.

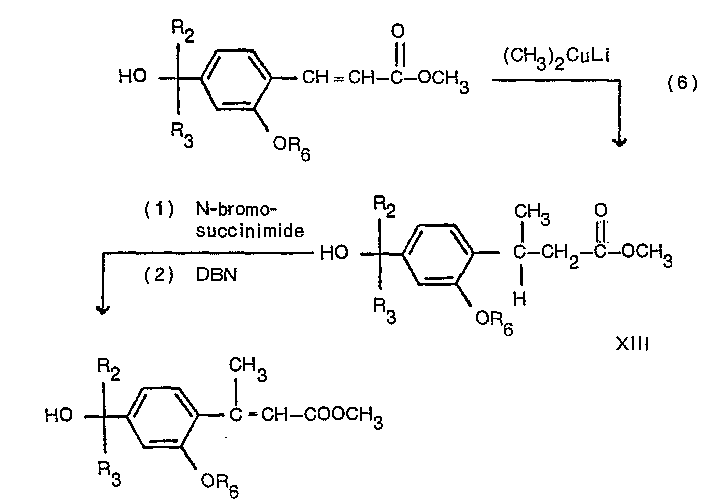

A compound of formula XII in which R₇ is hydrogen is contacted with methanol to obtain the methyl ester thereof which is then treated with dimethylcopperlithium by using the method of H.O. House and M. J. Umen, J. Org. Chem., 38, 3893 (1973) to give a compound of formula XIII. The compound of formula XIII is contacted with N-bromosuccinimide in a suitable solvent, such as carbon tetrachloride, in the presence of a suitable catalyst, such as benzoyl peroxide to obtain a reaction product which is then dehydrohalogenated with DBN to give the desired compound of the invention.

2-Alkoxy-4-polyhalohydroxyisopropyl phenylacetic acids of formula I are prepared by a process described by equation (7). 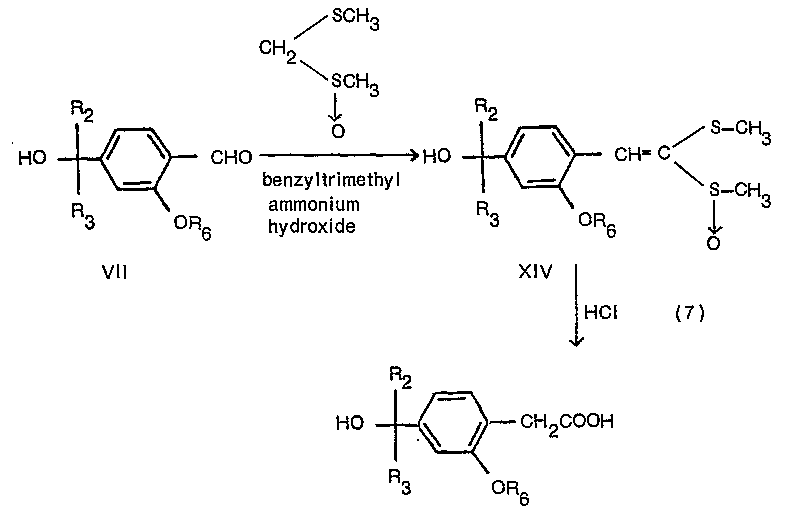 A compound of formula VII is contacted with methyl methylthiomethyl sulfoxide in a suitable solvent, such as tetrahydrofuran and in the presence of a suitable catalyst, such as benzyltrimethylammonium hydroxide, to give a compound of formula XIV which is then treated with HCI in a suitable solvent, such as dimethoxyethane, to hydrolyze the compound and to obtain a compound of the invention.

When compounds of formula I prepared by any of the processes of the invention are acids, they can be converted into esters, thiolesters and N,N-dimethyl amides of the invention in the usual manner via the acid chloride (compounds of formula I wherein R₄=Cl). When the compound prepared by any of the processes of the invention is an ester, it can be converted into the corresponding acid by hydrolysis in the usual manner. Esters wherein R, is an acyl group are prepared from esters of formula I where R₁ is H by reaction with acid chlorides or anhydrides with or without solvents. Because of the tertiary nature and high acidity of the alcohol group, esterfication is rather slow at room temperature but can be greatly accelerated by using high boiling solvents (with or without the addition of a base) or using refluxing pyridine as a solvent and base.

The invention is further illustrated by the following examples in which all temperatures are given in degrees C and all percentages are by weight unless otherwise stated. As used herein "consisting essentially of" has its customary meaning, i.e., it does not exclude unspecified conditions or materials which do not prevent the advantages of the invention from being realized.

### Example 1

### 4-[2,2,2-Trifluoro-1-hydroxy-1-(trifluoromethyl)ethyl]benzenepropanoic Acid

To 250 ml of dichloromethane are added 267 g (1.5 mole) of ethyl benzene propionate to obtain a solution which is added to a 1 liter stainless steel reactor equipped with gas inlets having shut off valves. To this solution are added 240 g (1.8 mole) of alumimum chloride. The reactor is sealed, cooled to -40°, and evacuated. Then to the reactor are added 249 g (1.5 mole) of hexafluoroacetone and the bomb is again sealed. The reactor is shaken, allowed to warm up to 25-30⁰, and then shaken at this temperature for 8-12 hours. The reactor is vented and the resulting reaction mixture is decanted. The reaction mixture is added to 1 liter of ice and 250 ml of concentrated hydrochloric acid to obtain an aqueous mixture which is then extracted thoroughly with dichloromethane. The resulting dichloromethane solution is washed with water, dried with anhydrous magnesium sulfate, filtered and evaporated at reduced pressure to obtain a residual oil.

This residual oil is heated with 1 N NaOH on a steam bath for 3 hours and is then cooled and acidified. Extraction with ether and then evaporation give a residual solid which is then recrystallized from chlorobutane to give 160 g of 4-[2,2,2-trifluoro-1-hydroxy-1-(trifluoromethyl)ethyl]benzenepropanoic acid having a melting point of 79°-81 °.

### Examples 2-6

The procedure of Example 1 can be used with the appropriate alkylaryl ester and polyhaloketone to give the indicated product.

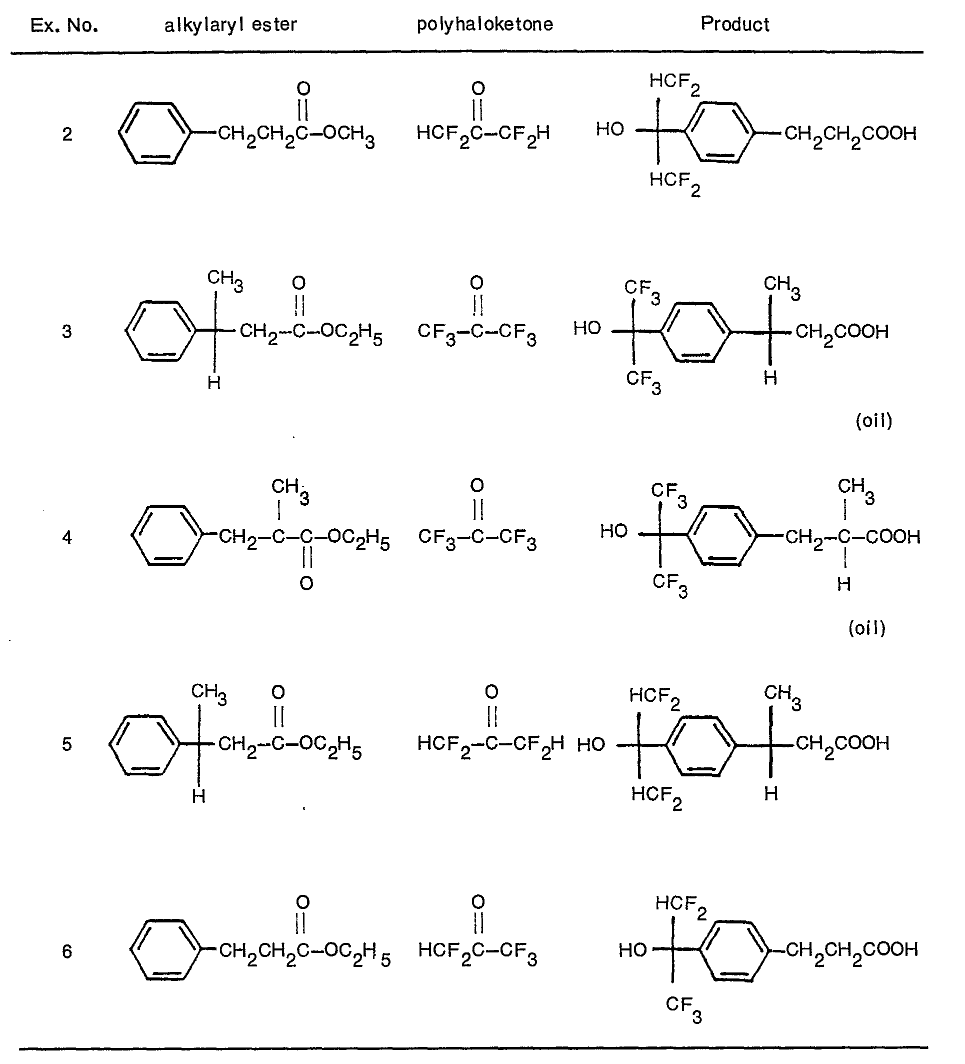

### Example 7

### 3-{4-[2,2,2-Trifluoro-1-hydroxy-1-(trifluoromethyl)ethyl]phenyl}-2-propenoic Acid.

### A: 4-[1-(Acetyloxy)-2,2,2-trifluoro-1-(trifluoromethyl)ethyl]benzaldehyde.

To a solution of 156 g (0.60 mole) of 4-[2,2,2-trifluoro-1-hydroxy-1-(trifluoromethyl)ethyl]-toluene in 675 ml of acetic anhydride are added 135 ml of concentrated sulfuric acid at 0°. To the solution stirred at 0° is added a solution of 168 g (1.68 mole) of chromium trioxide in 750 ml of acetic anhydride dropwise over a two-hour period. After stirring is continued for an additional two hours, the resulting reaction mixture is poured into five liters of ice and is diluted with five liters of water to obtain an aqueous mixture which is allowed to stand for 18 hours. An oily solid with water above it is obtained. Water is decanted from the oily solid. The solid is dissolved in ether to obtain a solution which is washed with water and sodium bicarbonate solution, dried with anhydrous magnesium sulfate, filtered and evaporated at reduced pressure to give a oily solid. The solid is triturated with ether, filtered and recrystallized from chlorobutane to give 60 g of α,α-bis-acetyloxy-4-[1-(acetyloxy)-2,2,2-trifluoro-1-(trifluoromethyl)ethyljtoluene, m.p. 93-94.6⁰.

To 130 ml of ethanol are added 60 g (0.15 mole) of α,α-bis-acetyloxy-4-[1-(acetyloxy)-2,2,2-tri- fluoro-1-(trifluoromethyl)ethyl]toluene and 13.5 ml of concentrated sulfuric acid in 135 ml of water. The resulting solution is stirred and heated at reflux for thirty minutes. The solution is then cooled and extracted with 1 liter of ether to obtain an ether solution which is washed with water and a saturated sodium bicarbonate solution until the washes were basic. The ether solution is dried with anhydrous magnesium sulfate, filtered and evaporated at reduced pressure to give 42 g of 4-[1-(acetyloxy)-2,2,2-trifluoro-1-(trifluoromethyl)ethyl]benzaldehyde. This compound was not further purified but was used directly in the next step.

### B: 3-{4-[1-(Acetyloxy)-2,2,2-trifluoro-1-(trifluoromethyl)ethyl]phenyl} 2-propenoic acid.

To 29 ml of 95% ethanol are added 35 g (0.11 mole) of the compound whose preparation is described in part A, 12.5 g (0.11 mole) of malonic acid, and 2.8 ml of pyridine. The resulting solution is stirred, heated at reflux for 18 hours, and then is evaporated at reduced pressure to obtain a residual oil which is taken up in ether. The resulting ether solution is washed with 1 N hydrochloric acid, dried with anhydrous magnesium sulfate, filtered and evaporated at reduced pressure to give a solid which is recrystallized from a benzene-hexane mixed solvent to give 30 g of 3-{4-[1-(acetyloxy)-2,2,2-trifluoro-1-(trifluoromethyl)ethyl]phenyl}-2-propenoic acid having a melting point of 144―147°

### C: 3-{4-[2,2,2-Trifluoro-1-hydroxy-1-(trifluoromethyl) ethyl]phenyl}-2-propenoic acid.

To 100 ml of water are added 8 g (0.02 mole) of the compound whose preparation is given in part B and 2 g (0.05 mole) of sodium hydroxide to obtain a reaction mixture which is stirred and heated at reflux for 18 hours. The solution is then cooled and made acid with concentrated hydrochloric acid to obtain a precipitate which is extracted with ether. The resulting ether solution is dried with anhydrous magnesium sulfate, filtered, and evaporated at reduced pressure to give a residual solid which is recrystallized from chlorobutane to give 5.5 g of 3-{4-[2,2,2-trifluoro-1-hydroxy-1-(trifluoromethyl)-ethyl]phenyl{ 2-propenoic acid having a melting point of 186-188⁰.

### Examples 8-11

The procedure of Example 7 can be used with the appropriate substituted benzaldehyde and malonic acid to give the indicated product.

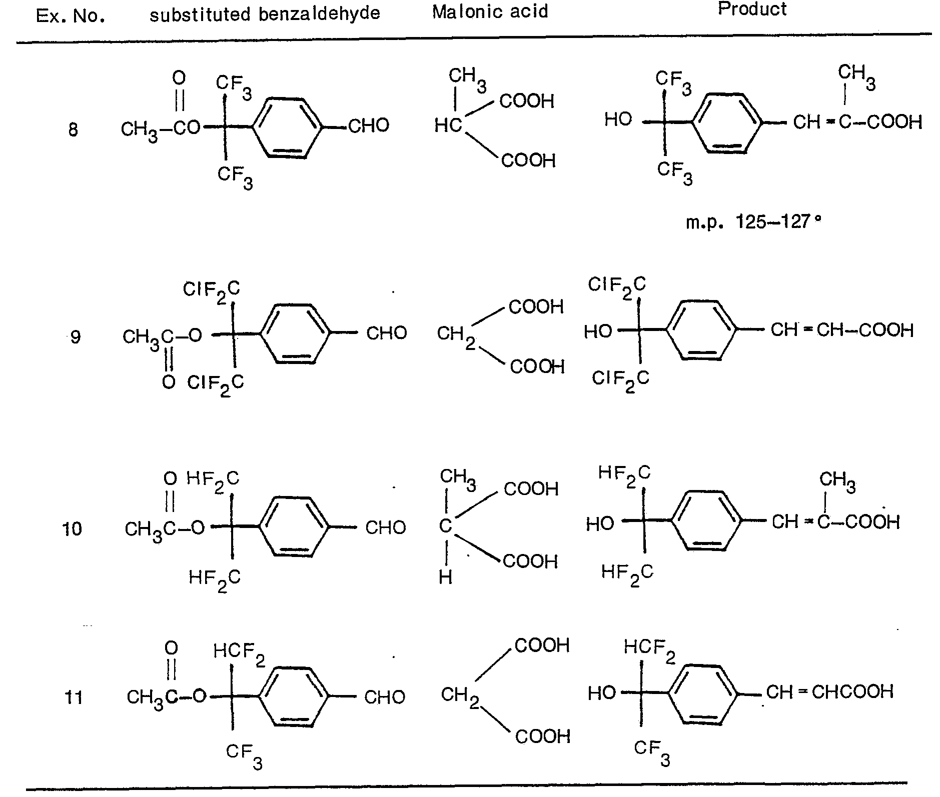

### Example 12

### 3-Methyl-3-{4-[2,2,2-trifluoro-1-hydroxy-1-(trifluoromethyl)ethyl]phenyl} 2-propenoic Acid.

### This compound can be prepared by the following preparation.

### A: Methyl 3-Bromo-3-methyl-{4-[2,2,2-trifluoro-1-hydroxy-1-(trifluoromethyl)ethyl]phenyl}propanoate.

To a solution of methyl 3-methyl-3-{4-[2,2,2-trifluoro-1-hydroxy-1-(trifluoromethyl)ethyl]phenyl}- propanoate, which can be prepared in a manner similar to that described in Example 46, in carbon tetrachloride can be added N-bromosuccinimide and a small quantity of benzoyl peroxide. The resulting mixture can be stirred and heated at reflux until thin layer chromatographic analysis of a reaction sample indicates the reaction to be complete. The succinimide can be removed by filtration and the solvent removed at reduced pressure to give methyl 3-bromo-3-methy)-(4-[2,2,2-triftuoro-1-hydroxy-1-(trifluoromethyl)ethyl]phenyl} propanoate.

### B: 3-Methyl-3-{4-[2,2,2-trifluoro-1-hydroxy-1-(trifluoromethyl)ethyl]phenyl}2-propenoic Acid

To a solution of methyl 3-bromo-3-methyl-{4-[2,2,2-trifluoro-1-hydroxy-1-(trifluoromethyl)ethyl]-phenyl} propanoate in tetrahydrofuran can be added an equivalent amount of 1,5-diazabicyclo[3.4.0]nonene-5. The resulting reaction mixture can be stirred at elevated temperature until thin layer chromatographic analysis of a reaction sample indicates the reaction to be complete. The solvent can be removed and the residual oil dissolved in ether to obtain an ether solution which can be extracted with 1 N hydrochloric acid and then by 2 N sodium hydroxide solution. The resulting sodium hydroxide solution can be heated on the steam bath, cooled and made acidic with concentrated hydrochloric acid to obtain a product which can be isolated from the aqueous phase with ether. The resulting ether solution can be dried with anhydrous magnesium sulfate, filtered, and evaporated to give 3-methyl-3-{4-[2,2,2-trifluoro-1-hydroxy-1-(trifluoromethyl)ethyl]phenyl}-2-propenoic acid.

### Examples 13-15

The procedure of Example 12 can be used with the appropriate substituted ester to give the indicated product.

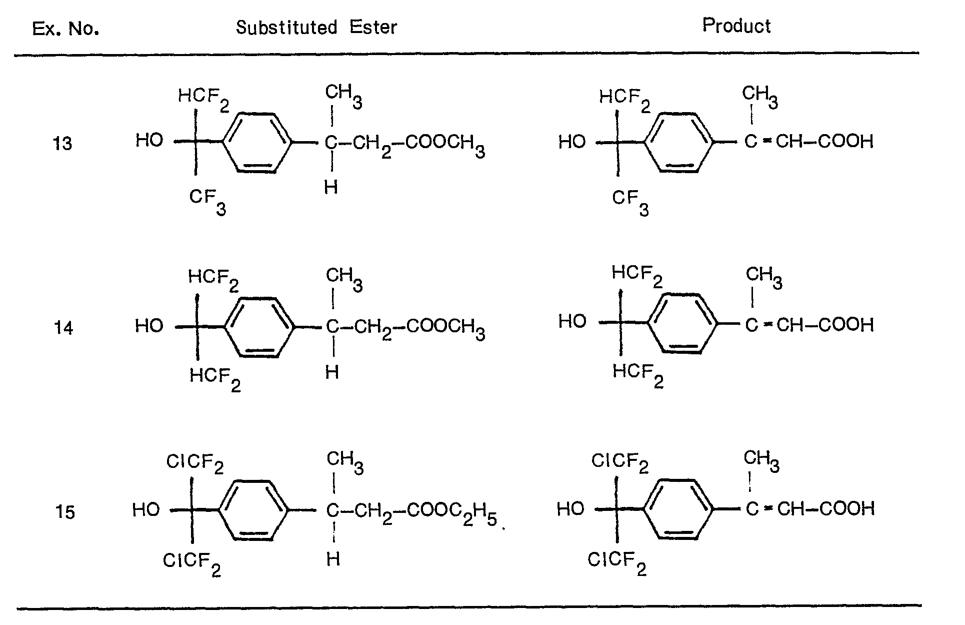

### Example 16

### 2-Nitro-4-[2,2,2-trifluoro-1-hydroxy-1-(trifluoromethyl)ethyl]benzenepropanoic Acid.

To a solution of 63.2 g (0.20 mole) of 4-[2,2,2-trifluoro-1-hydroxy-1-(trifluoromethyl)-ethyl]benzenepropanoic acid in 160 ml of concentrated sulfuric acid are added 40 ml of fuming nitric acid (90%) dropwise with stirring at 0―5°. Following the nitric acid addition the solution is allowed to warm to 20° with stirring over a 1-hr period. The solution is then added to a mixture of ice and water and allowed to stand for 18 hours. A precipitate results and is filtered, washed with water and dissolved in ether to obtain a solution which is washed with water, dried with anhydrous magnesium sulfate, filtered, and evaporated to give a residual solid. The residual solid is recrystallized from chlorobutane to give 40 g of 2-nitro-4-[2,2,2-trifluoro-1-hydroxy-1-(trifluoromethyl)ethyl]benzenepropanoic acid having a melting point of 115-116¹.

### Examples 17-19

The procedure of Example 16 can be used with the appropriate aralkyl acid to give the indicated product. The 4[2,2,2-trifluoro-1-hydroxy-1-(trifluoromethyl)ethyl]benzeneacetic acid given as a starting material in Example 17 can be prepared by reacting the corresponding alkylaryl ester with hexafluoroacetone using a procedure similar to that given in Example 1.

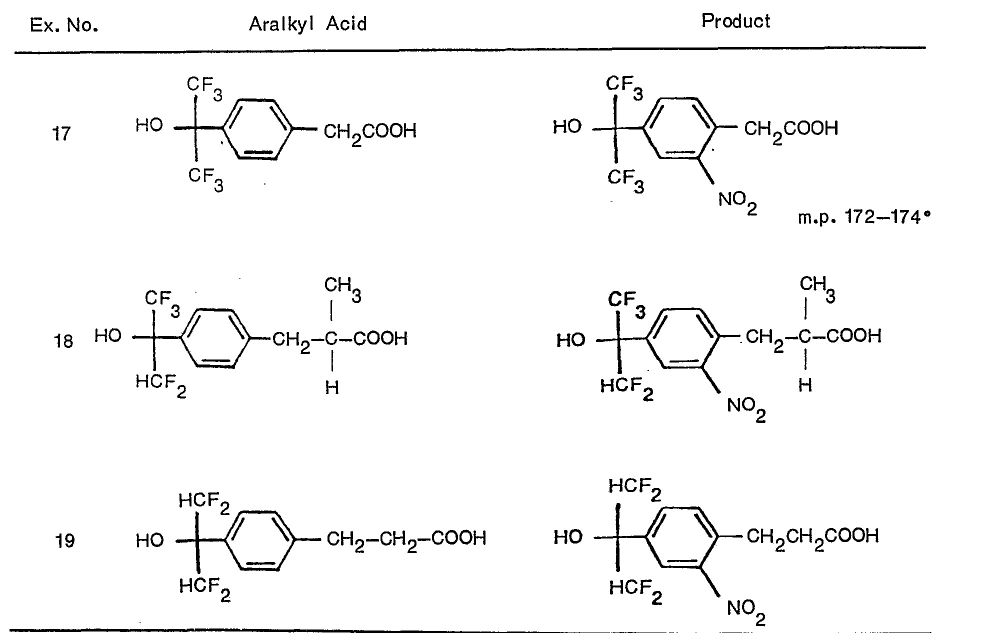

### Examples 20-23

The procedure of Example 12 can be used with the indicated 2-nitro-benzenepropanoic acid ester to give the indicated product.

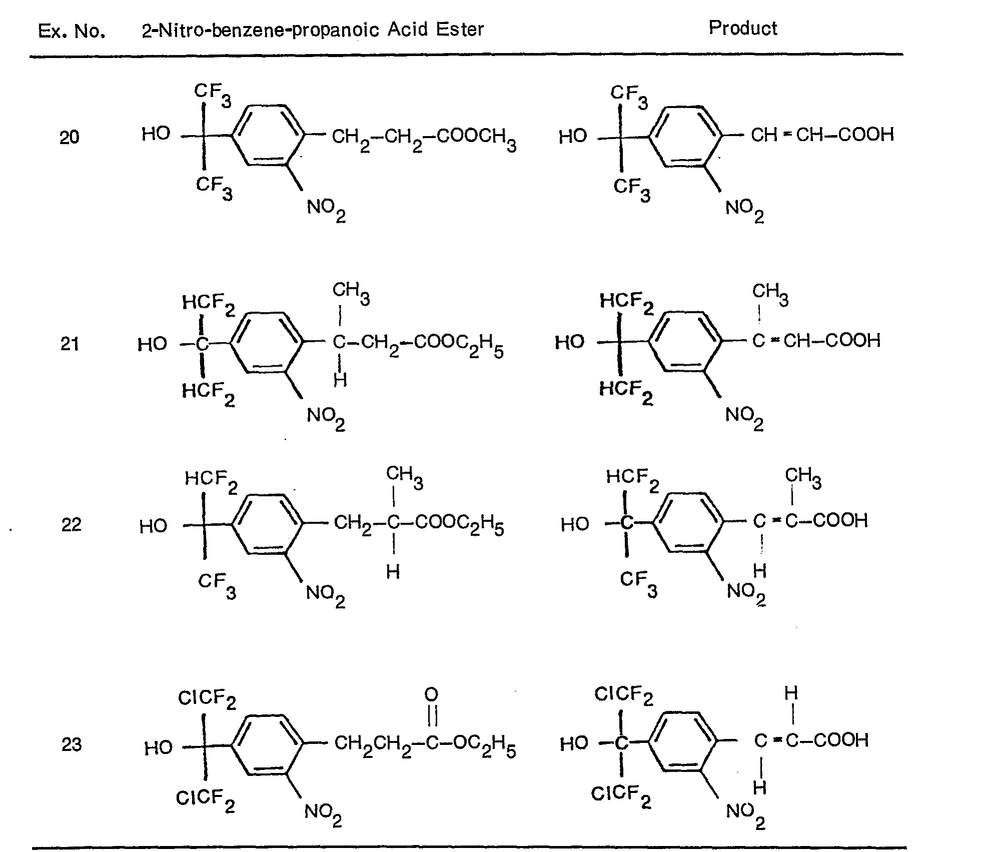

### Example 24

### 2-Methylthio-4-[2,2,2-trifluoro-1-hydroxy-1-(trifluoromethyl)ethyl]benzenepropanoic Acid

### This compound can be obtained by the following preparation.

### A: 3,4-Dihydro-7-[2,2,2-trifluoro-1-hydroxy-1-(trifluoromethyl)ethyl]-2H-1-benzothiapyran-2-one

To excess chlorosulfonic acid cooled with an ice-acetone bath can be added methyl 4-[2,2,2-tri- fluoro-1-hydroxy-1-(trifluoromethyl)ethyl]benzenepropanoate with stirring. The solution should be stirred for several hours. Addition of the reaction mixture to ice and isolation of the product will give 2-chlorosulfonyl-4-[2,2,2-trifluoro-1-hydroxy-1-(trifluoromethyl)ethyl]benzenepropanoic acid, methyl ester. Reduction of 2-chlorosulfonyl-4-[2,2,2-trifluoro-1-hydroxy-1-(trifluoromethyl)ethyl]benzenepropanoic acid, methyl ester with zinc in acetic acid followed by isolation of the product will give 2-mercapto-4-[2,2,2-trifluoro-1-hydroxy-1-(trifluoromethyl)ethyl]benzenepropanoic acid, methyl ester.

Heating 2-mercapto-4-[2,2,2-trifluoro-1-hydroxy -(trifluoromethyl)ethyl]benzenepropanoic acid, ethyl ester with p-toluenesulfonic acid in toluene at 'lux will give 3,4-dihydro-7-[2,2,2-trifluoro-1-hydroxy-1-(trifluoromethyl)ethyl]-2H-1-benzothiapyran-2-one.

### B: 2-Methylthio-4-[2,2,2-trifluoro-1-hydroxy-1-(trifluoromethyl)ethyl]benzenepropanoic Acid

To a solution of 3,4-dihydro-7-[2,2,2-trifluoro-1-hydroxy-1-(trifluoromethyl)ethyl]-2H-1-benzo- thiapyran-2-one in dimethylacetamide can be added a one molar excess of sodium hydroxide in methanol. The resulting solution should be heated and a one molar excess of methyl iodide should be added with stirring. From the resulting reaction mixture can be isolated 2-methylthio-4-[2,2,2-tri- fluoro-1-hydroxy-1-(trifluoromethyl)ethyl]benzenepropanoic acid, methyl ester. This ester should be dissolved in an aqueous solution of sodium hydroxide to obtain a solution which will be heated on a steam bath. Concentrated hydrochloric acid can then be added to this solution, thereby yielding a precipitate which can be isolated to give 2-methylthio-4-[2,2,2-trifluoro-1-hydroxy-1-(trifluoromethyl)ethyl]benzenepropanoic acid.

### Examples 25-28

The procedure of Example 24 can be used with the appropriate aralkyl ester to give the indicated benzothiapyran-2-one which can be reacted with the appropriate alkyl halide to give indicated product.

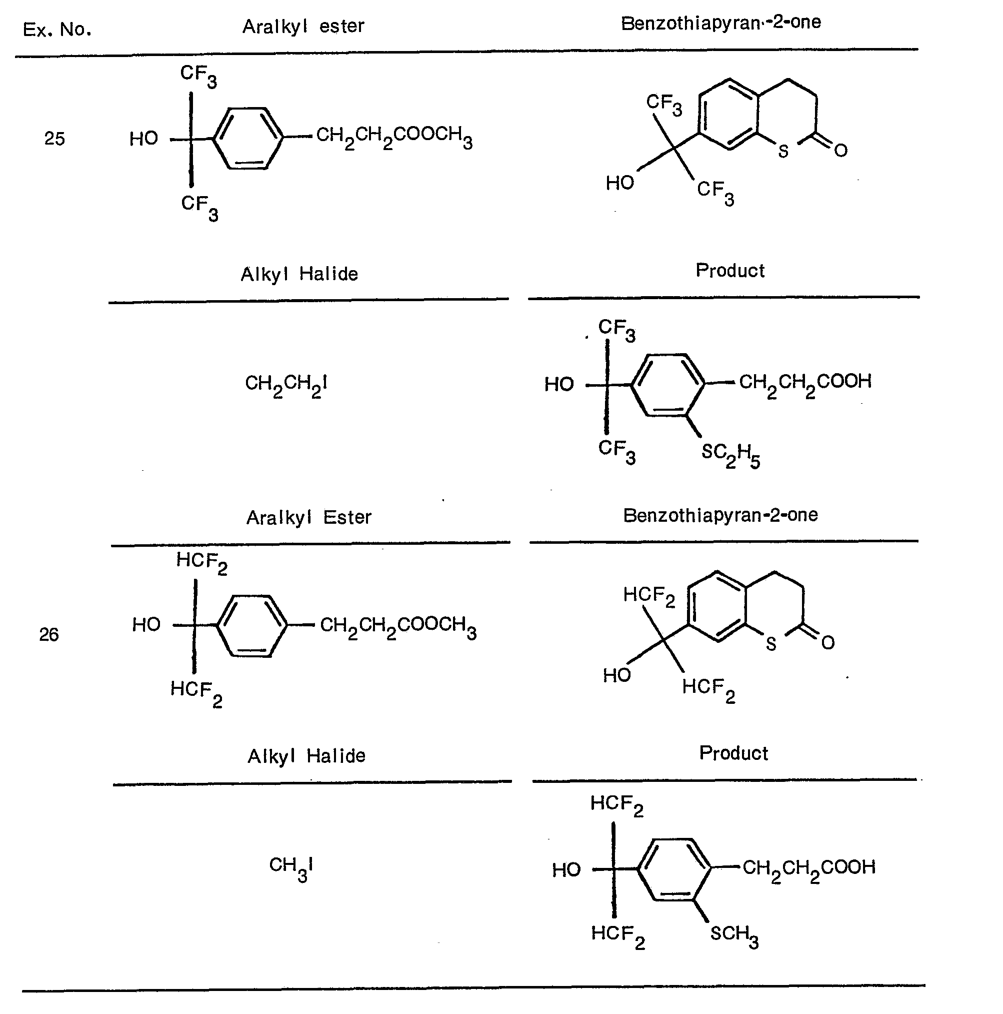

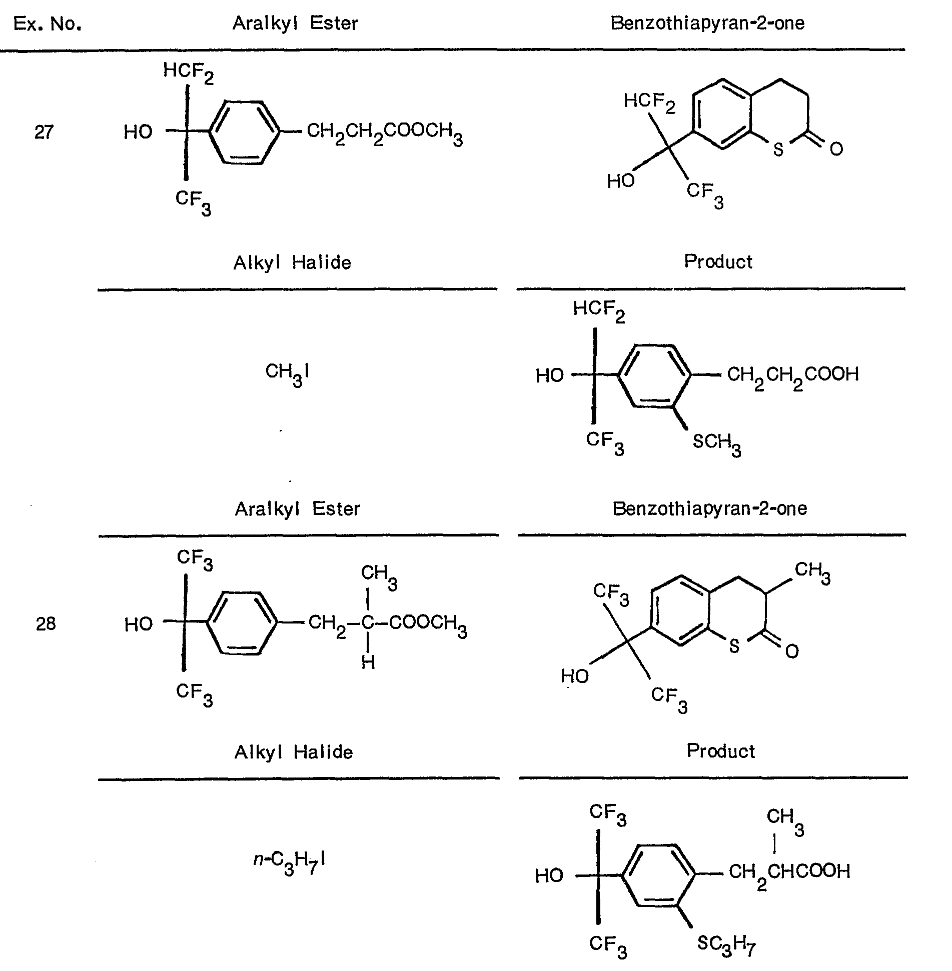

### Example 29

### 3-{2-Methoxy-4-[2,2,2-trifluoro-1-hydroxy-1-(trifluoromethyl)ethyl]phenyl}-2-propenoic Acid.

### A: 1-Methoxy-3-[2,2,2-trifluoro-1-hydroxy-1-(trifluoromethyl)ethyl]benzene

To a suspension of 13.5 g (0.55 g atoms) of sublimed magnesium chips in 25 ml of anhydrous tetrahydrofuran in a reaction flask fitted with a water-cooled condenser are added 10 g (0.05 mole) of 3-bromo-l-methoxybenzene and 0.5 ml of 1,2-dibromoethane. The resulting reaction mixture is stirred and heated gently until a reaction is initiated. To the stirred reaction mixture is added dropwise a solution of 90 g (0.48 mole) of 3-bromo-1-methoxybenzene in 250 ml of anhydrous tetrahydrofuran at such a rate that gentle reflux is maintained. The reaction mixture is stirred for an additional two hours. The resulting solution is then cooled to 0° and the reaction flask is fitted with a jacketed dropping funnel topped by a dry ice condenser. The water condenser is replaced by an additional dry ice condenser. To the jacketed dropping funnel are added 85 g (50 ml) (0.55 mole) of condensed hexafluoroacetone. The condensed hexafluoroacetone is added dropwise to the stirred solution which is cooled to 0 to -10¹ with an ice-acetone bath. When the addition of the hexafluoroacetone is complete, the resulting solution is allowed to warm to 20-25⁰ and stirring is continued for an additional 18 hours. The solution is cooled to 0° and treated with 6N hydrochloric acid to obtain an acidic solution to which is added 1-liter of ether. The resulting ether layer is washed with water, washed with a 5% sodium bicarbonate solution, dried with anhydrous magnesium sulfate, filtered and evaporated at reduced pressure to obtain a residual solid which is recrystallized from chlorobutane to give 78 g of 1-methoxy-3-[2,2,2-trifluoro-1-hydroxy-1-(trifluoromethyl)ethyl]benzene.

### B: 2-Methoxy-4-[2,2,2-trifluoro-1-hydroxy-1-(trifluoromethyl)ethyl]benzaldehyde

To solution of 31.6 g (0.12 mole) of 1-methoxy-3-[2,2,2-trifluoro-1-hydroxy-1-(trifluoromethyl)-ethyl]benzene in 200 ml dichloromethane are added 38 g (0.2 mole) of titanium tetrachloride at 0―5° with stirring under a nitrogen atmosphere. After the resulting solution is stirred for 15 minutes, 11.5 g (0.1 mole) of α,α-dichloromethyl ether are added dropwise at 0-5°C. Stirring is continued while the temperature of the solution is allowed to reach 20―25° during 18 hours. The solution is then added with stirring to a solution of 100 ml 6N hydrochloric acid in 500 ml of ice and water. The resulting organic phase is separated leaving an aqueous phase which is washed with three 300 ml portions of dichloromethane. The combined dichloromethane extracts are washed with 1 N hydrochloric acid and then with saturated sodium chloride solution. The dichloromethane solution is dried with anhydrous magnesium sulfate, filtered, and evaporated at reduced pressure to obtain an oily solid which is triturated with chlorobutane, filtered and recrystallized from dichlorobutane to give 14.5 g of 2-methoxy-4-[2,2,2-trifluoro-1-hydroxy-1-(trifluoromethyl)ethyl]benzaldehyde having a melting point of 144-1460.

### C: 3-{2-Methoxy-4-[2,2,2-trifluoro-1-hydroxy-1-(trifluoromethyl)ethyl]phenyl}-2-propenoic Acid

To a solution of 15 g (0.05 mole) 2-methoxy-4-[2,2,2-trifluoro-1-hydroxy-1-(trifluoromethyl)-ethyl]benzaldehyde in 50 ml of pyridine are added 11 g (0.11 mole) of malonic acid and 1 g of piperidine. The solution is stirred and gradually heated to 80°. Heating and stirring are continued for 1 hour at 80°. The solution is then heated at reflux for an additional 3 hours, cooled, and evaporated at reduced pressure to obtain a residual oil which is extracted into ether. The resulting ether solution is washed with water and 1 N hydrochloric acid, dried with anhydrous magnesium sulfate, filtered, and evaporated at reduced pressure to obtain a residual solid which is recrystallized from chlorobutane/hexane mixed solvent to give 10.5 g of 3-{2-methoxy-4-[2,2,2-trifluoro-1-hydroxy-1-(trifluoromethyl)ethyl]phenyl}-2-propenoic acid having a melting point of 138-140⁰.

### Examples 30-35

The procedures of Example 29 can be used with the appropriate starting materials to give the indicated products.

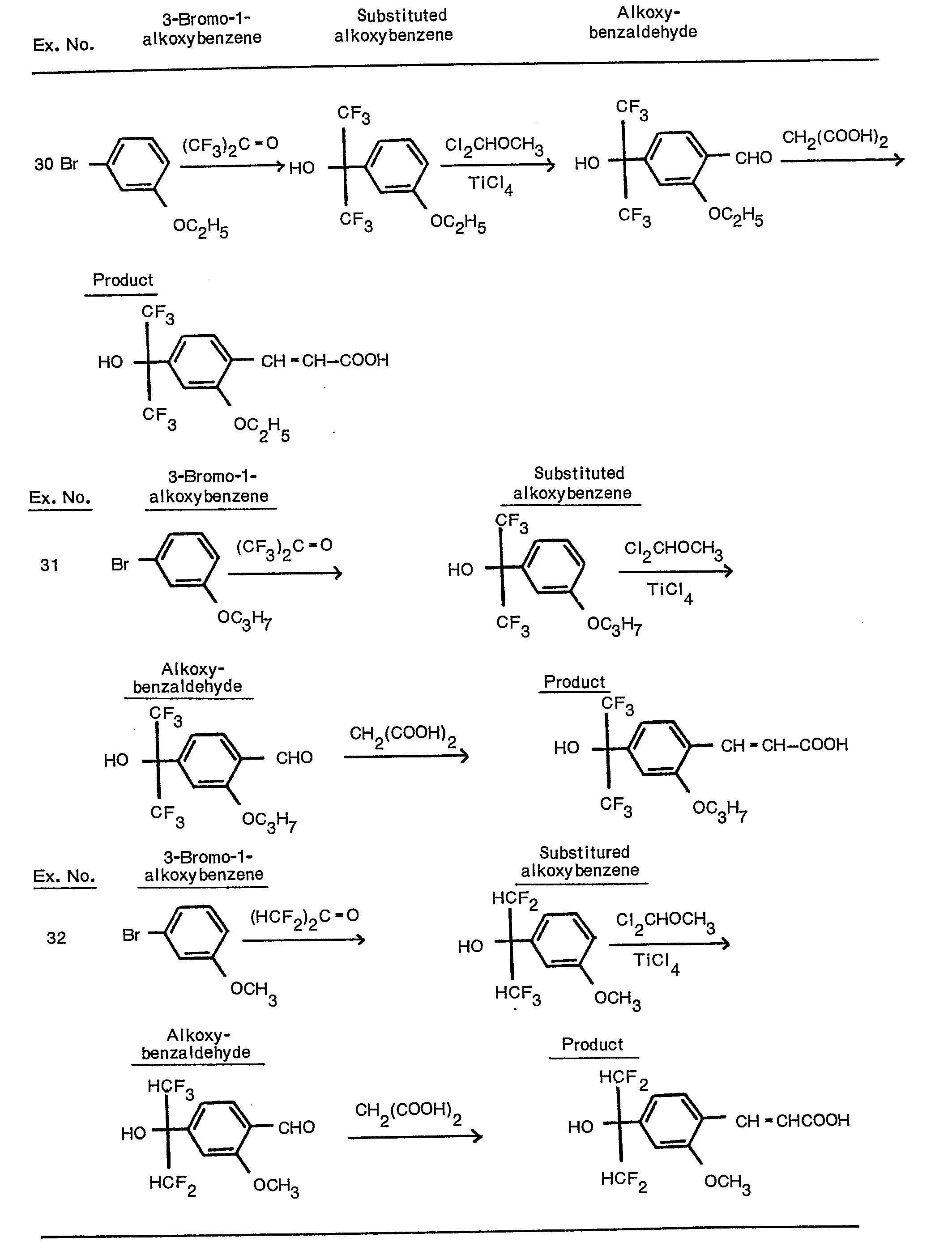

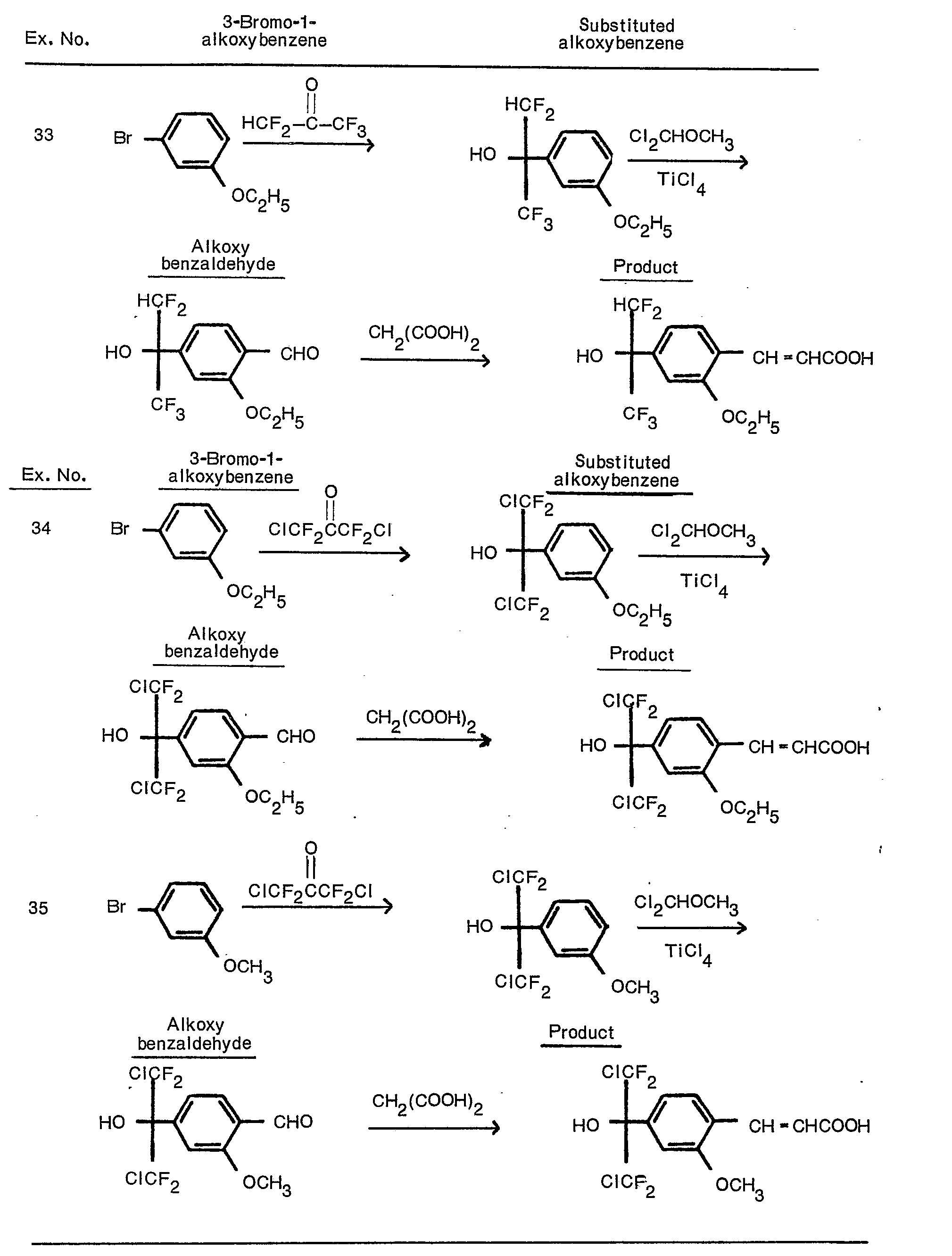

### Example 36

### 2-Methoxy-4-[2,2,2-trifluoro-1-hydroxy-1-(trifluoromethyl)ethyl]benzenepropanoic Acid

To 100 ml of ethanol are added 8.8 g (0.025 mole) of 3-{2methoxy-4-[2,2,2-trifluoro-1-hydroxy-1-(trifluoromethyllethyl]phenyl}-2-propenoic acid to produce a solution which is added to a pressure bottle and flushed with nitrogen. To the solution is added 1 g of 10% palladium on carbon. The pressure bottle is placed in a hydrogenation apparatus and pressurized to 1384 g/cm² (50 Ibs/in.²) with hydrogen. The pressure bottle is shaken until the pressure ceases dropping. The resulting solution is vented, filtered and evaporated at reduced pressure to obtain a residual solid which is recrystallized from chlorobutane to give 7.0 gm of 2-methoxy-4-[2,2,2-trifluoro-1-hydroxy-1-(trifluoromethyl)-ethyl]benzenepropanoic acid having a melting point of 116―117°.

### Examples 37-41

The procedure of Example 36 can be used with the appropriate 2-alkoxy-phenylpropenoic acid to give the indicated product.

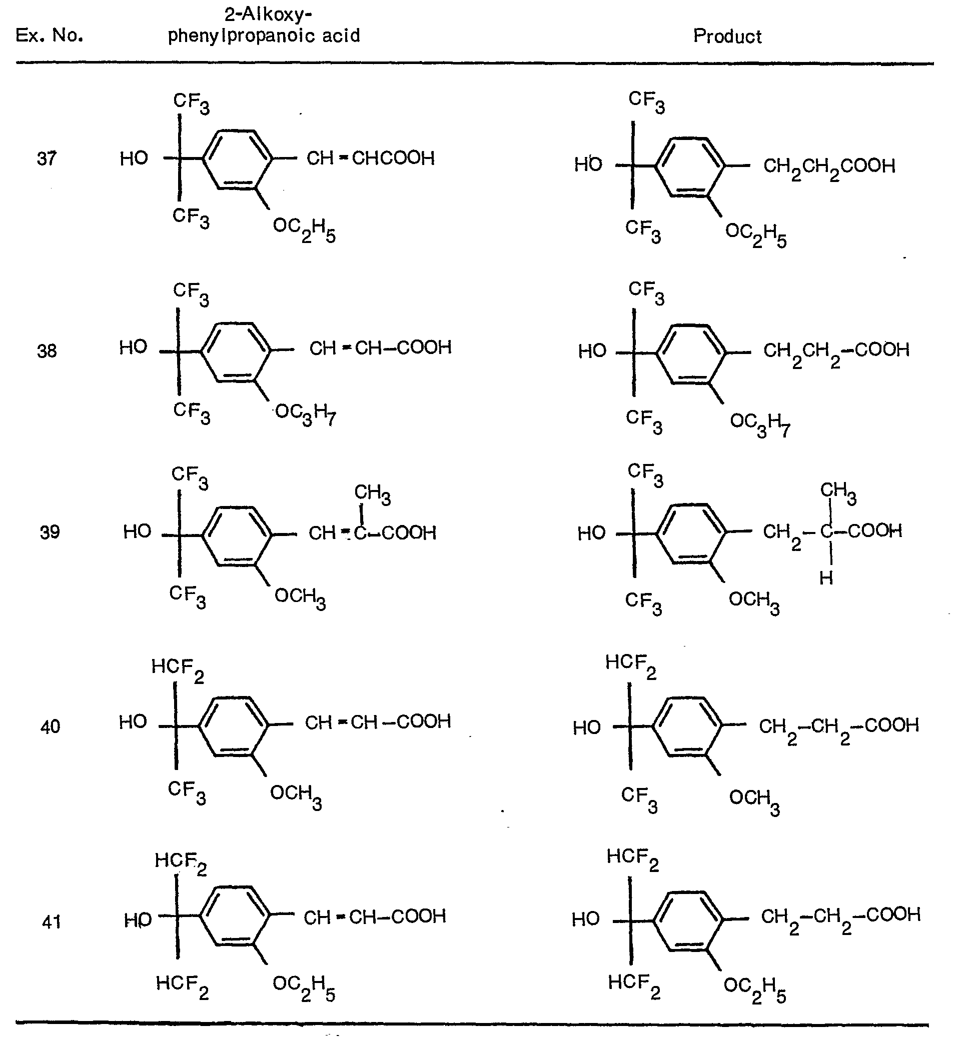

### Example 42

### 2-Methoxy-4-[2,2,2-trifluoro-1-hydroxy-1-(trifluoromethyl)ethyl]benzeneacetic Acid

To a solution of 20 g (0.066 mole) of 2-methoxy-4-[2,2,2-trifluoro-1-hydroxy-1-(trifluoromethyl)ethyl]benzaldehyde in 100 ml of anhydrous tetrahydrofuran are added 10 g (0.071 mole) of methyl methylthiomethyl sulfoxide and 50 ml of a 40% solution of benzyltrimethylammonium hydroxide in methanol. The resulting solution is stirred and heated at reflux for 96 hours. The solution is then concentrated at reduced pressure to obtain a residual oil which is next added to 50 ml of water and neutralized with 6N hydrochloric acid. The resultant mixture is extracted with ether, dried with anhydrous magnesium sulfate, filtered and evaporated at reduced pressure to give a residual oil which is purified by high pressure liquid chromatography to give 16 g of an oil shown to be homogeneous by thin-layer chromatography. The oil is added to a solution of 100 ml 6N hydrochloric acid in 100 ml dimethoxyethane to obtain a solution which is then stirred and heated at reflux for 20 hours. The solution is concentrated at reduced pressure to give a residual oil which is dissolved in 100 ml of 1 N sodium hydroxide solution. The solution is heated to 60° for 1 hour, neutralized with 1 N hydrochloric acid and extracted with ether to obtain an ether solution which is dried with anhydrous magnesium sulfate, filtered and evaporated at reduced pressure to give a residual solid. This residual solid is recrystallized from chlorobutane to give 10.2 g of 2-methoxy-4-[2,2,2-trifluoro-1-hydroxy-1-(trifluoromethyl)ethyl]benzeneacetic acid having a melting point of 133-135°.

### Examples 43-45

The procedure of Example 42 can be used with the appropriate 2-alkoxybenzaldehyde to give the indicated product.

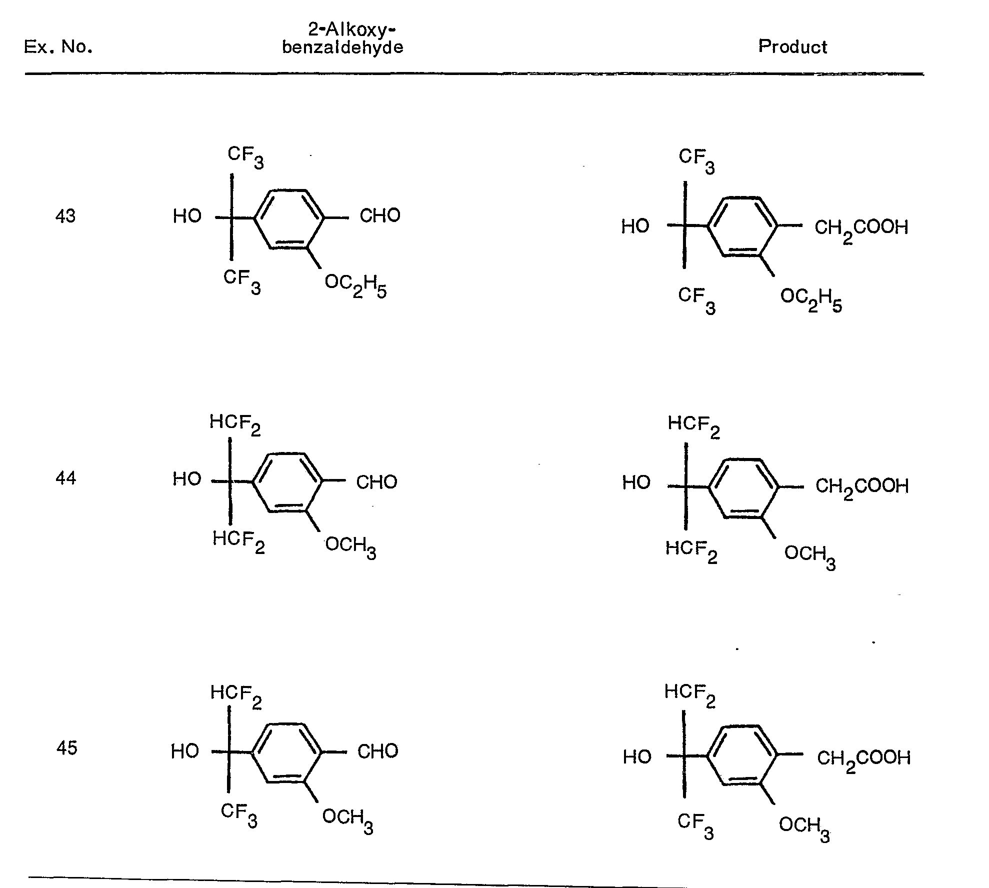

### Example 46

### Methyl 2-Methoxy-4-[2,2,2-trifluoro-1-hydroxy-1-(trifluoromethyl)ethyl]benzenepropanoate

To a solution of 50 ml of methanol in 50 ml toluene are added 7.0 g (0.02 mole) of 2-methoxy-4-[2,2,2-trifluoro-1-hydroxy-1-(trifluoromethyl)ethyl]benzenepropanoic acid and 1 ml of concentrated sulfuric acid. The resulting solution is stirred, heated at reflux for 18 hours, then concentrated to 20 ml at reduced pressure, and extracted with ether. The resulting ether solution is washed with 5% sodium bicarbonate solution and water. The solution is dried with anhydrous magnesium sulfate, filtered and concentrated at reduced pressure to obtain a residual solid which is recrystallized from petroleum ether to give 5.5 g of methyl 2-methoxy-4-[2,2,2-trifluoro-1-hydroxy-1-(trifluoromethyl)ethyl]benzene- propanoate having a melting point of 78-80^{0.}

### Examples 47-59

The procedure of Example 46 can be used with the appropriate aralkyl acid and alcohol to give the indicated product.

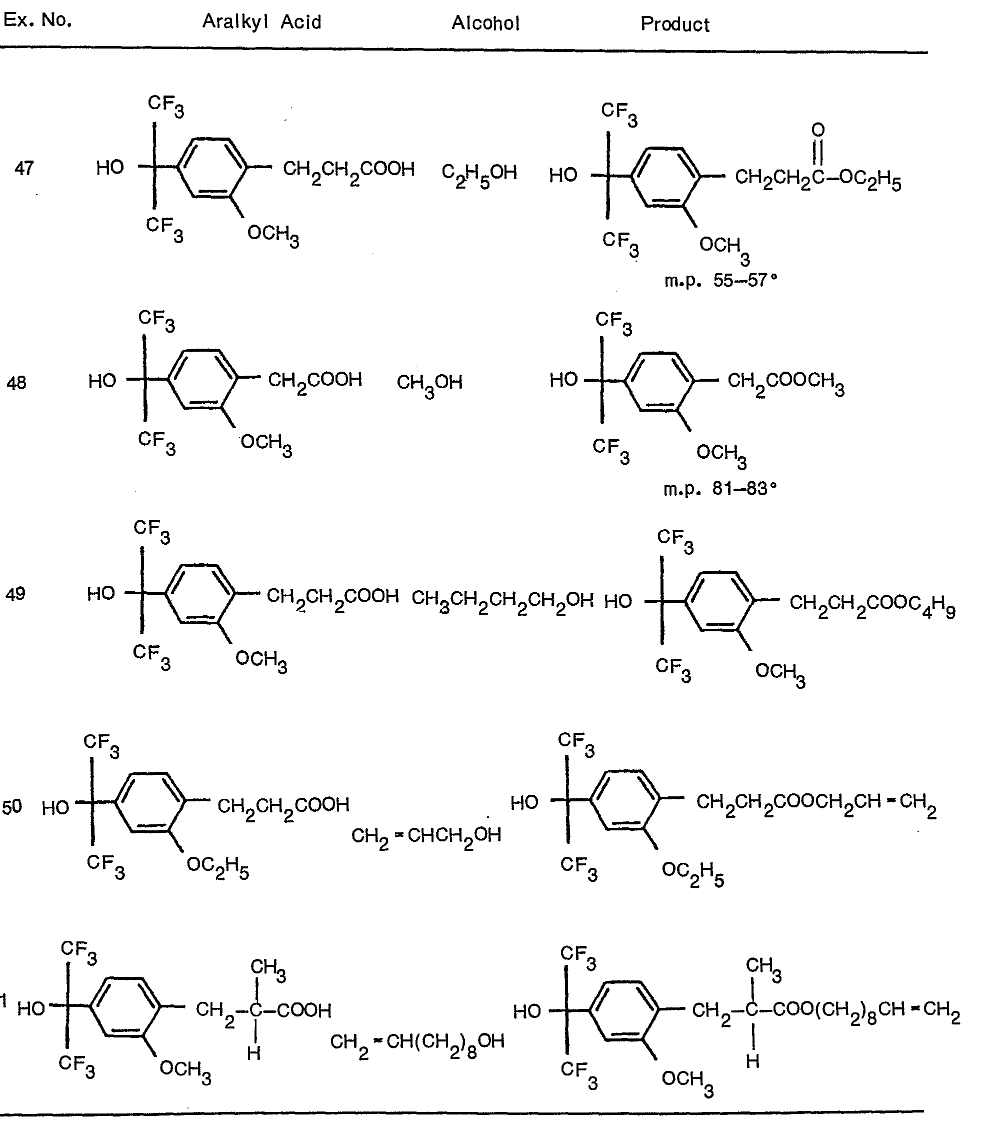

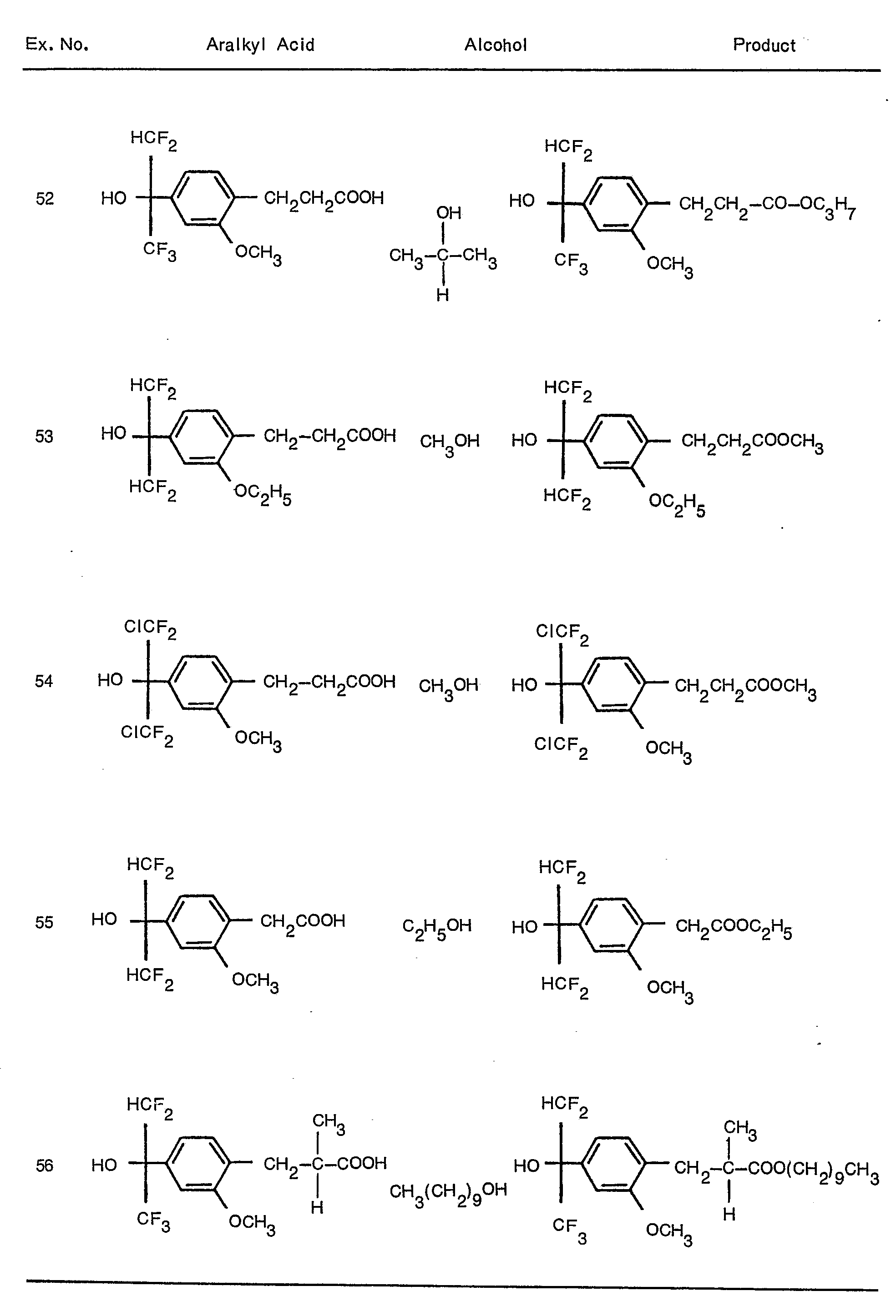

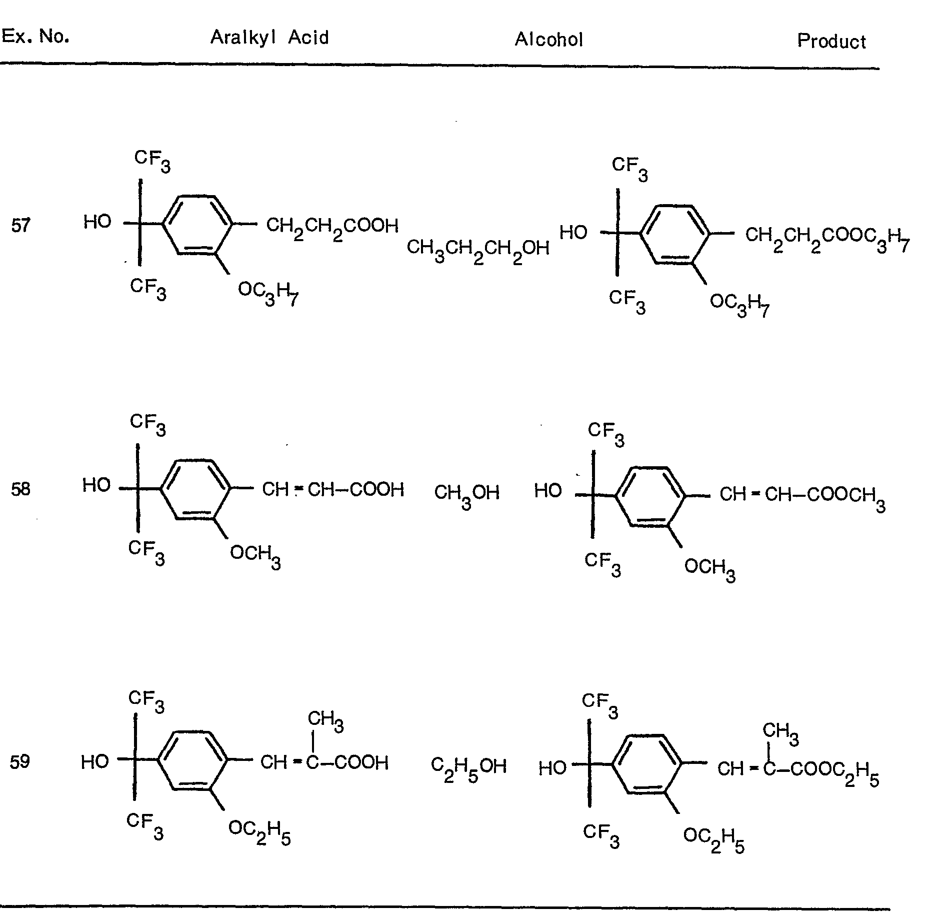

### Example 60

### 2-Methoxy-4-[2,2,2-trifluoro-1-hydroxy-1-(trifluoromethyl)ethyl]benzenepropanoic Acid, (4-Methoxyphenyl) Ester.

To a solution of 16 g (0.046 mole) of 2-methoxy-4-[2,2,2-trifluoro-1-hydroxy-1-(trifluoromethyl)ethyl]benzenepropanoic acid in 300 ml of toluene are added 8 gm (0.060 mole) of oxalyl chloride and 3 drops of dimethylformamide. After evolution of hydrogen chloride ceases the resulting reaction mixture is heated at reflux for 2 hours to obtain a solution which is cooled and concentrated at reduced pressure to give 2-methoxy-4-[2,2,2-trifluoro-1-hydroxy-1-(trifluoromethyl)ethyl]benzene- propanoyl chloride.

A solution of 5 g (0.22 mole) of 2-methoxy-4-[2,2,2-trifluoro-1-hydroxy-1-(trifluoromethyl)-ethyl]benzenepropanoyl chloride in 100 ml of toluene is added to a solution of 2.5 g (0.02 mole) of p-methoxyphenol and 3 g (0.03 mole) of triethylamine in 50 ml of toluene. The resulting solution is stirred and heated at reflux for 4 hours. The solution is then cooled and evaporated at reduced pressure to obtain a residual oil which is extracted with ether. The resulting ether solution is washed with 2N hydrochloric acid and 5% sodium bicarbonate solution, and dried with anhydrous magnesium sulfate. The resulting dried solution is filtered and concentrated at reduced pressure to obtain a residual oil which is chromatographed on silica (Silica¡@ CC-7 silica manufactured by Mallinckrodt, Inc.). Elution with toluene gives, after concentration, 2-methoxy-4-[2,2,2-trifluoro-1-hydroxy-1-(trifluoromethyl)-ethyl]benzenepropanoic acid, (4-methoxyphenyl) ester as a clear homogenous oil. The formation of the ester was confirmed by the presence of an ester carbonyl band at 5.79µ.

### Examples 61-71

The procedure of Example 60 can be used with the appropriate aralkyl acid and alcohol or thiol to give the indicated product.

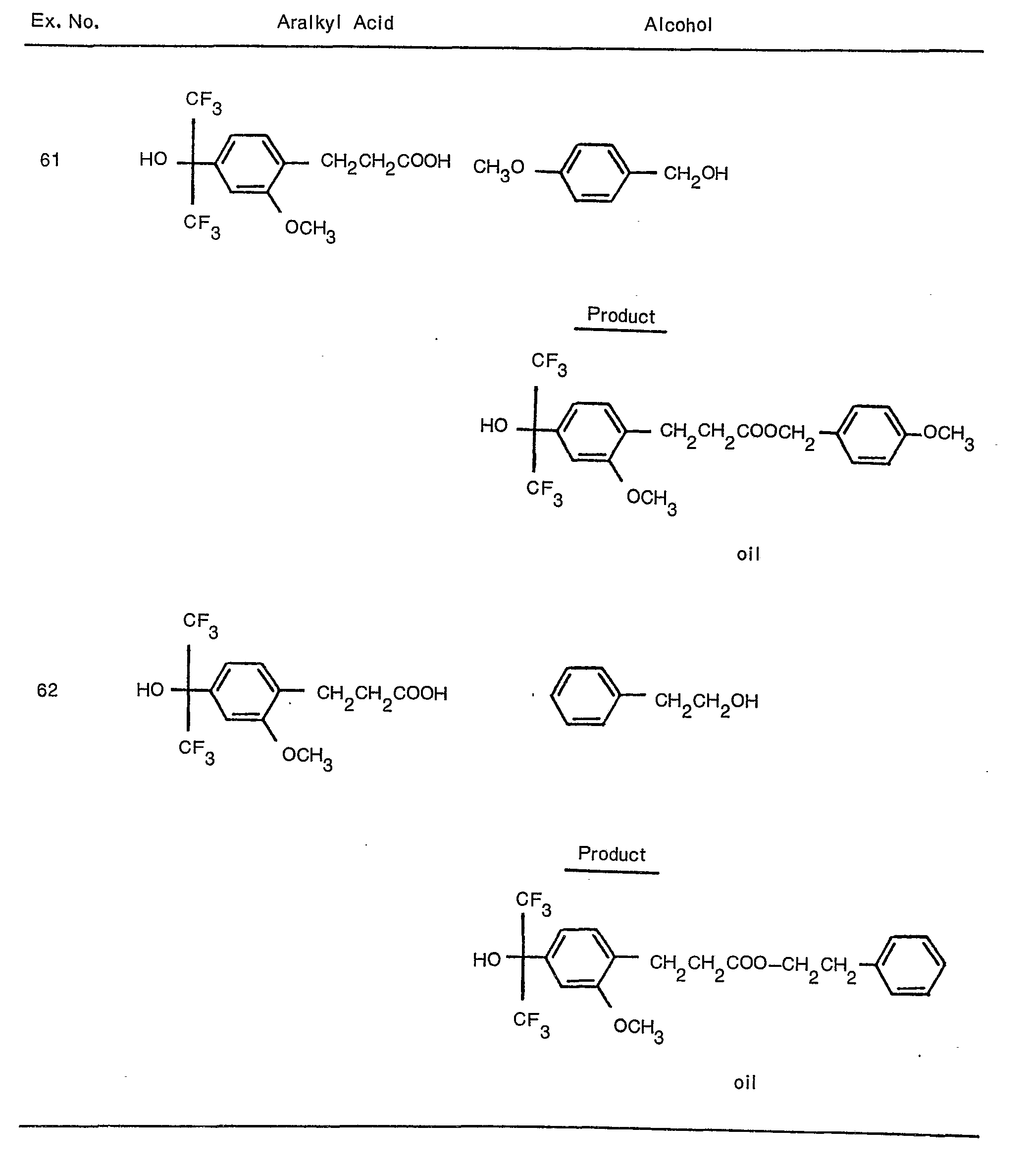

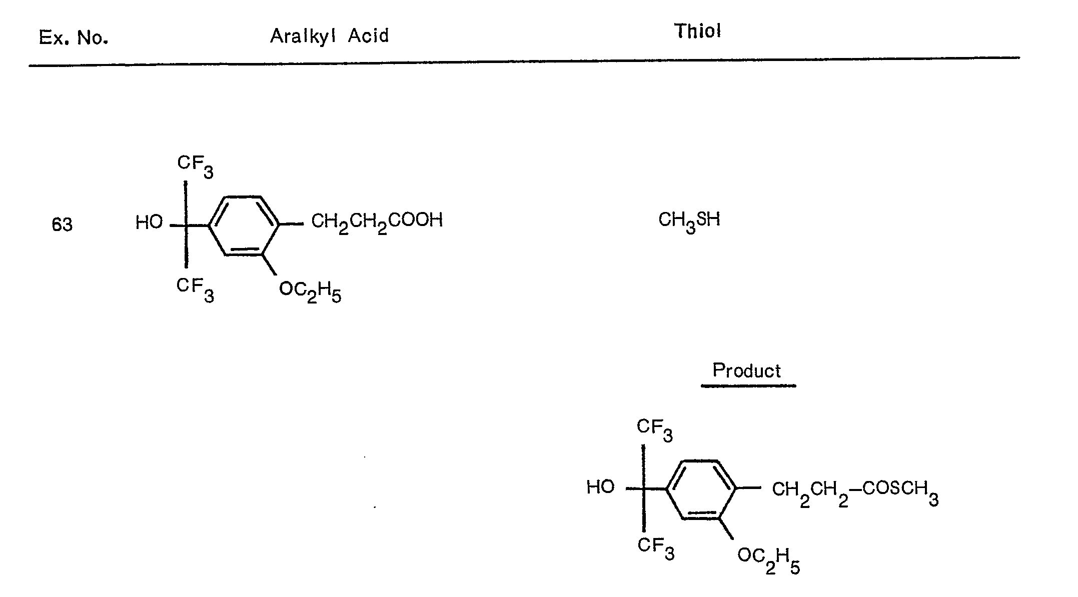

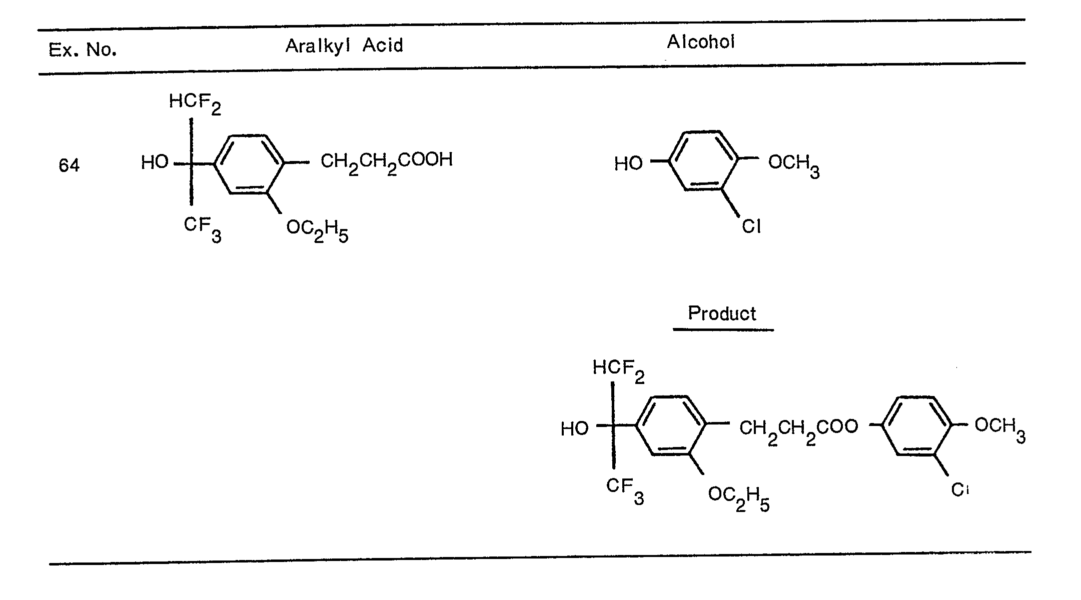

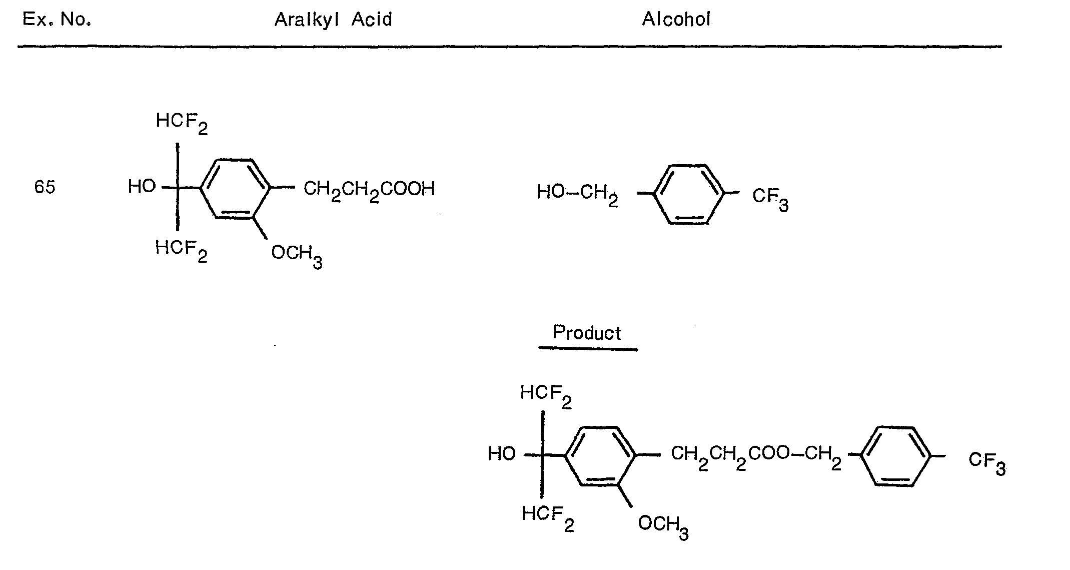

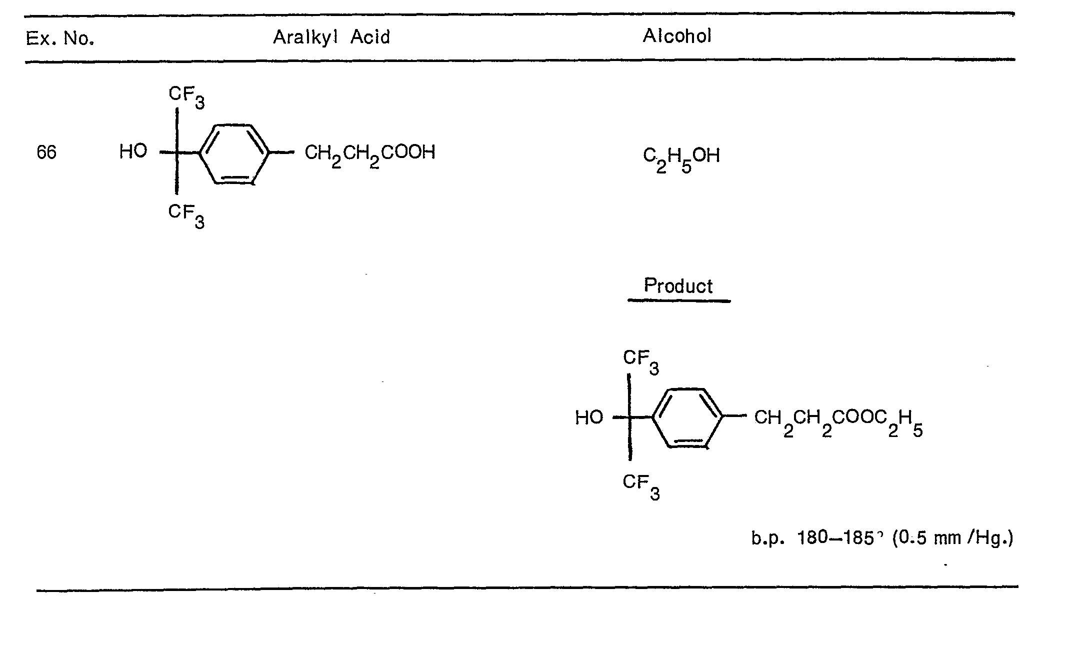

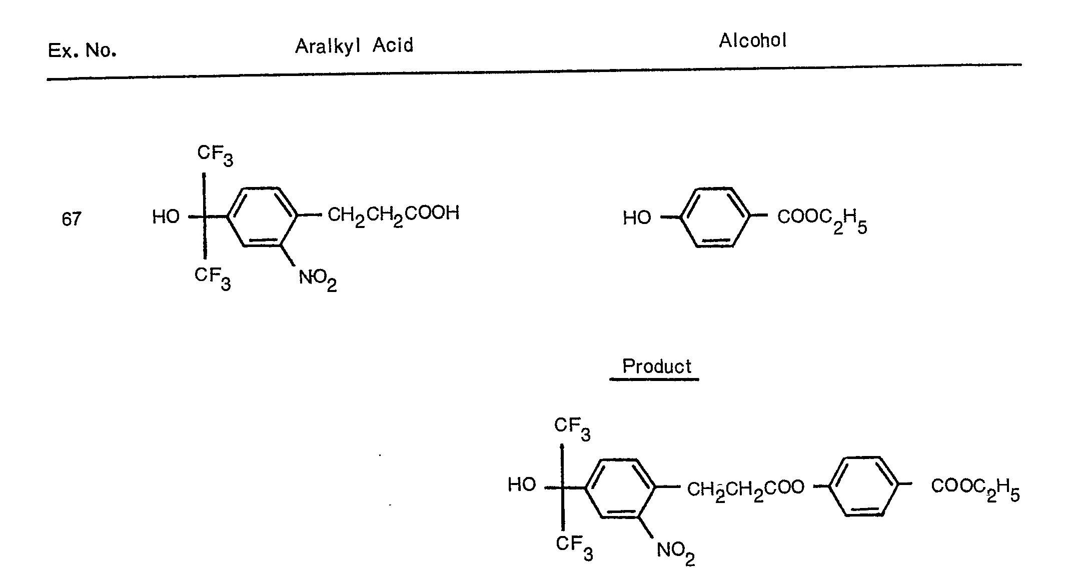

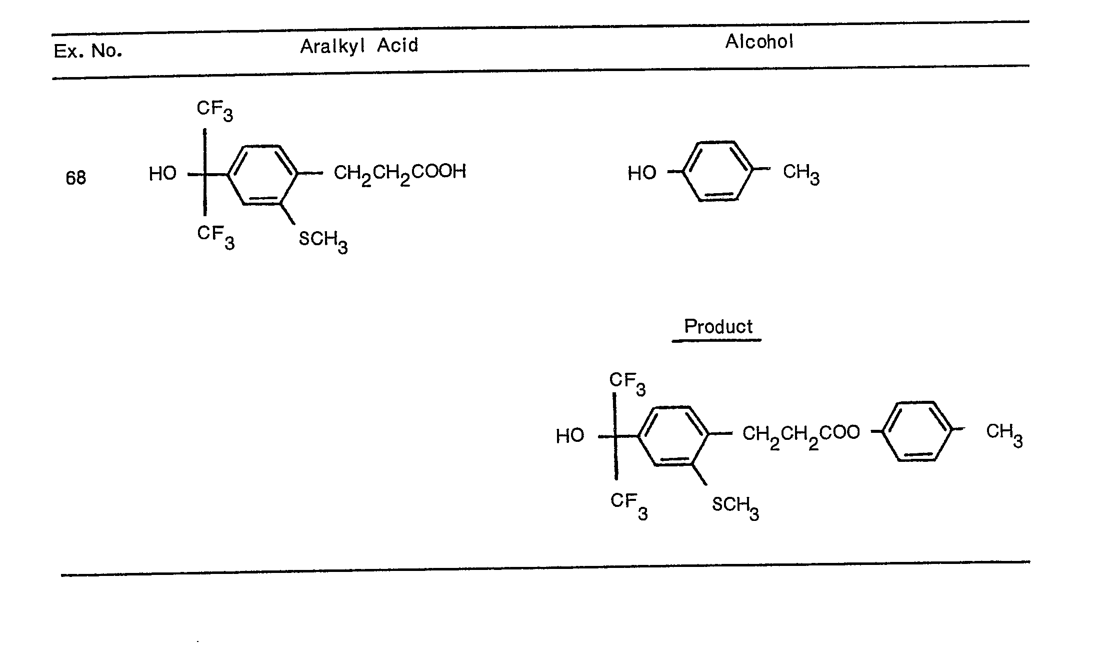

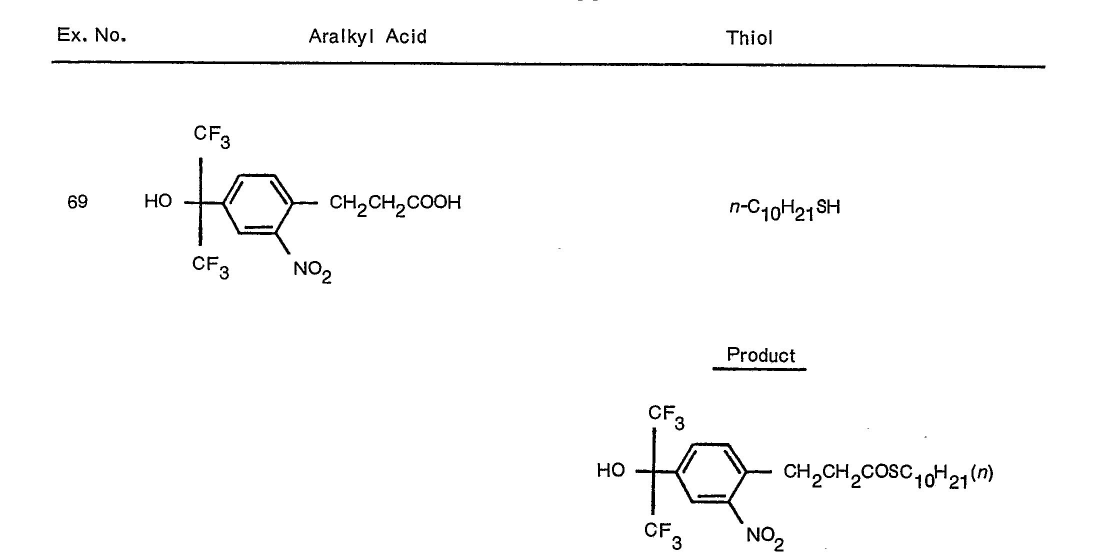

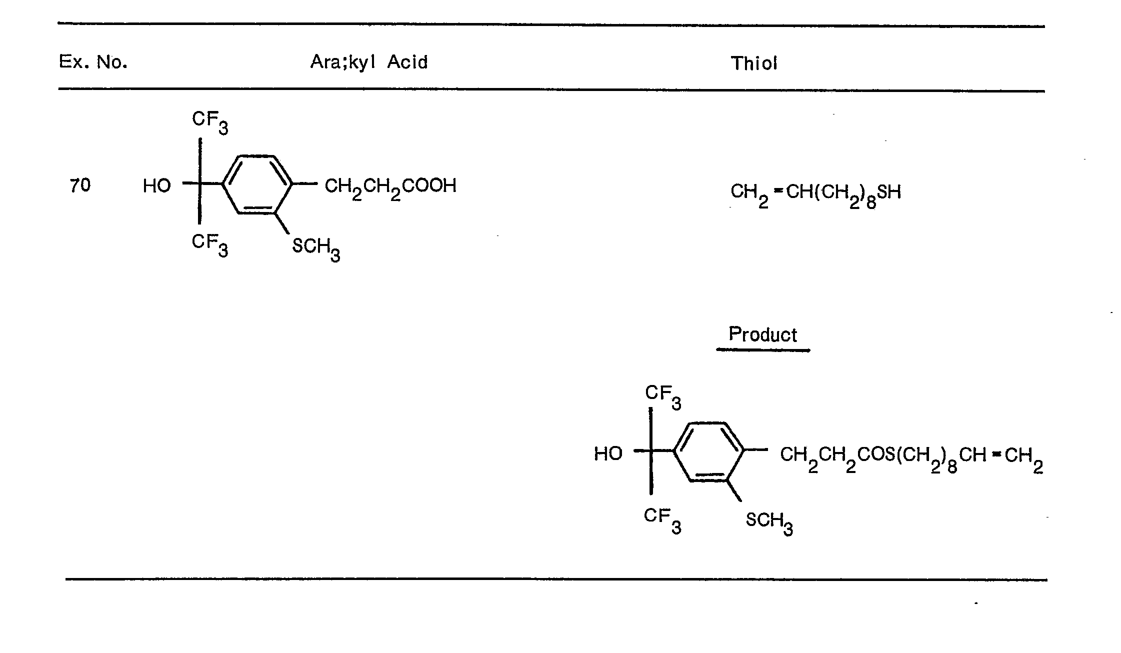

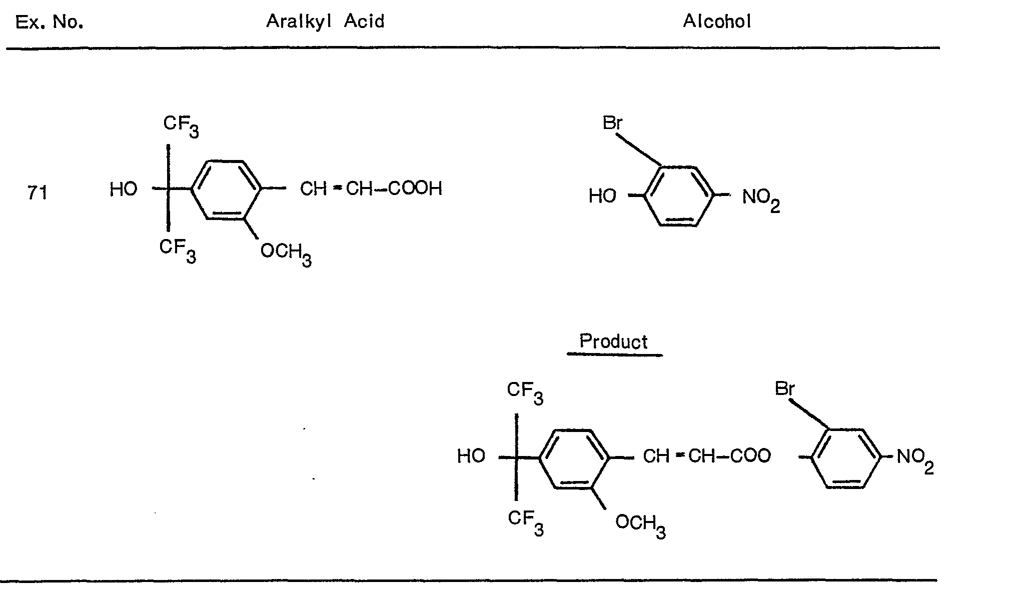

### Examples 72-79

The procedure of Example 60 can be used with the appropriate aralkyl acid and with dimethyl amine in place of an alcohol and triethylamine to give the indicated N,N-dimethylamide as the product.

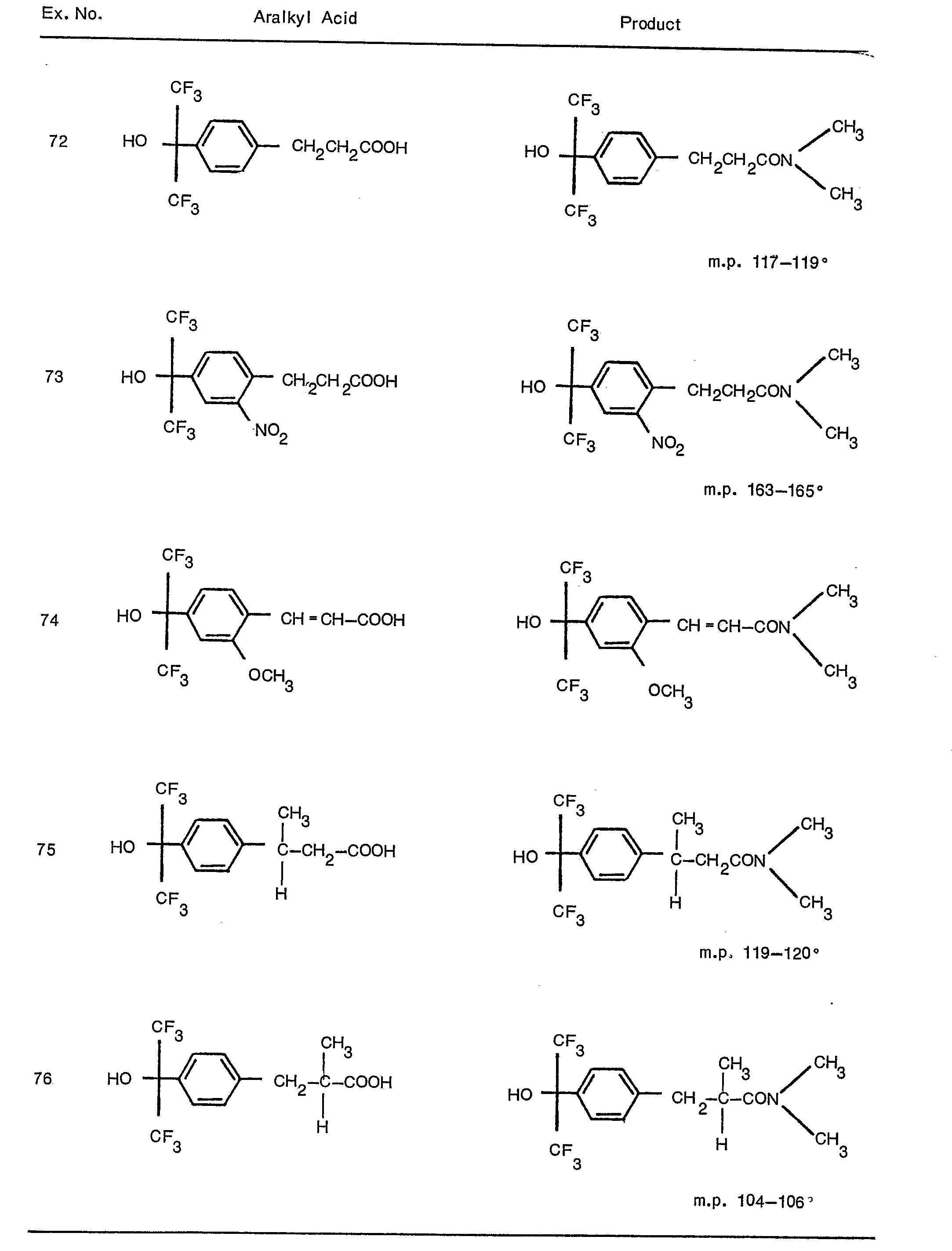

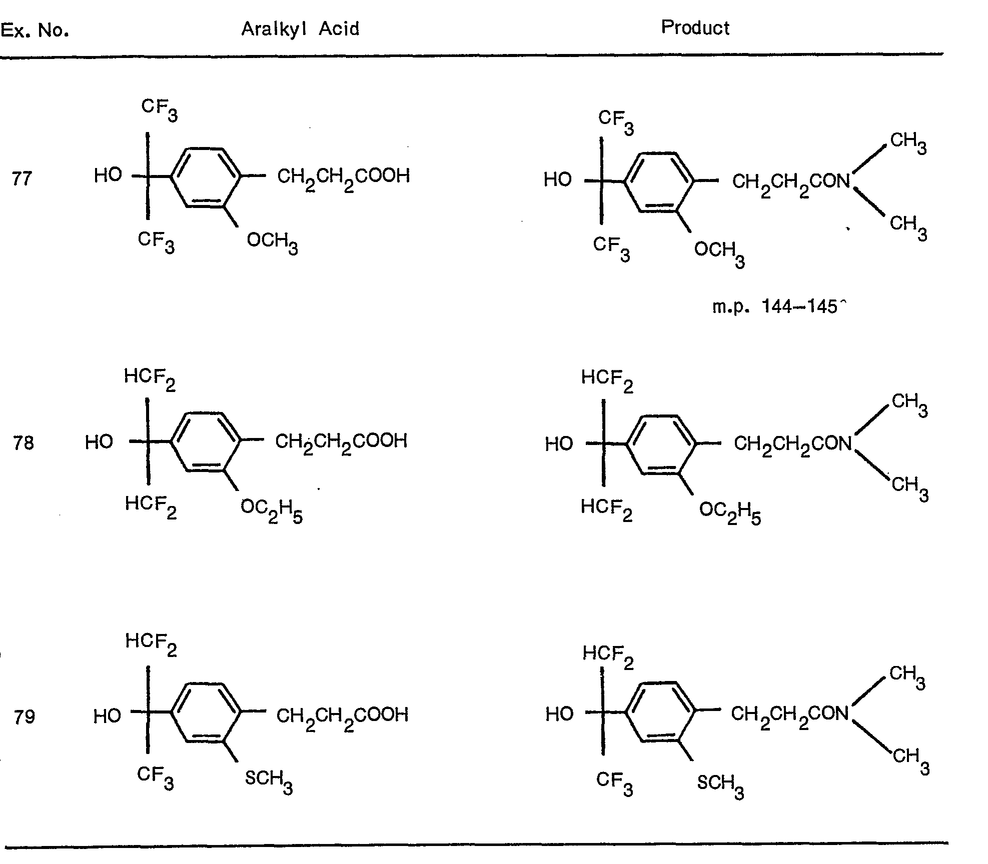

### Example 80

### 3-[2-Nitro-4-[2,2,2-trifluoro-1-methoxy-1-(trifluoromethyl)-ethyl]phenyl}-N,N-dimethylpropanamide

To a suspension of 2.15 g (0.085 mole) of sodium hydride in 200 ml dimethylformamide are added 29.1 g (0.075 mole) of 3-[2-nitro-4-[2,2,2-trifluoro-1-hydroxy-1-(trifluoromethyl)ethyl]phenyl}-N,N-dimethylpropanamide in 50 ml dimethylformamide at such a rate that excessive foaming is avoided. To the resulting suspension, stirred in a nitrogen atmosphere, are added 14.2 g (0.10 mole) of methyl iodide to give a reaction mixture which is stirred at room temperature overnight. The reaction mixture is then treated with 5 ml of ethanol, poured into 1-liter of water and extracted with ether. The resulting ether solution is washed with water, dried over anhydrous magnesium sulfate, filtered and evaporated at reduced pressure to obtain a residual oil which is chromatographed on Silicar® CC-7. Elution with chloroform gives a major fraction consisting of 16 g which is recrystallized from hexane to give 3-{2-nitro-4-[2,2,2-trifluoro-1-methoxy-1-(trifluoromethyl)ethyl]phenyl}-N,N-dimethylpropanamide having a melting point of 51-54°.

### Examples 81-86

The procedure of Example 80 can be used with the appropriate aralkyl ester or dimethylamide and alkyl halide or acyl halide to give the indicated product. When 1-hydroxy aralkyl acids are treated with alkyl halides as in Example 80 it is understood that the products are the corresponding 1-alkoxy aralkyl esters. The 1-alkoxy aralkyl acids indicated as products can be generated by basic hydrolysis of the corresponding 1-alkoxy aralkyl ester.

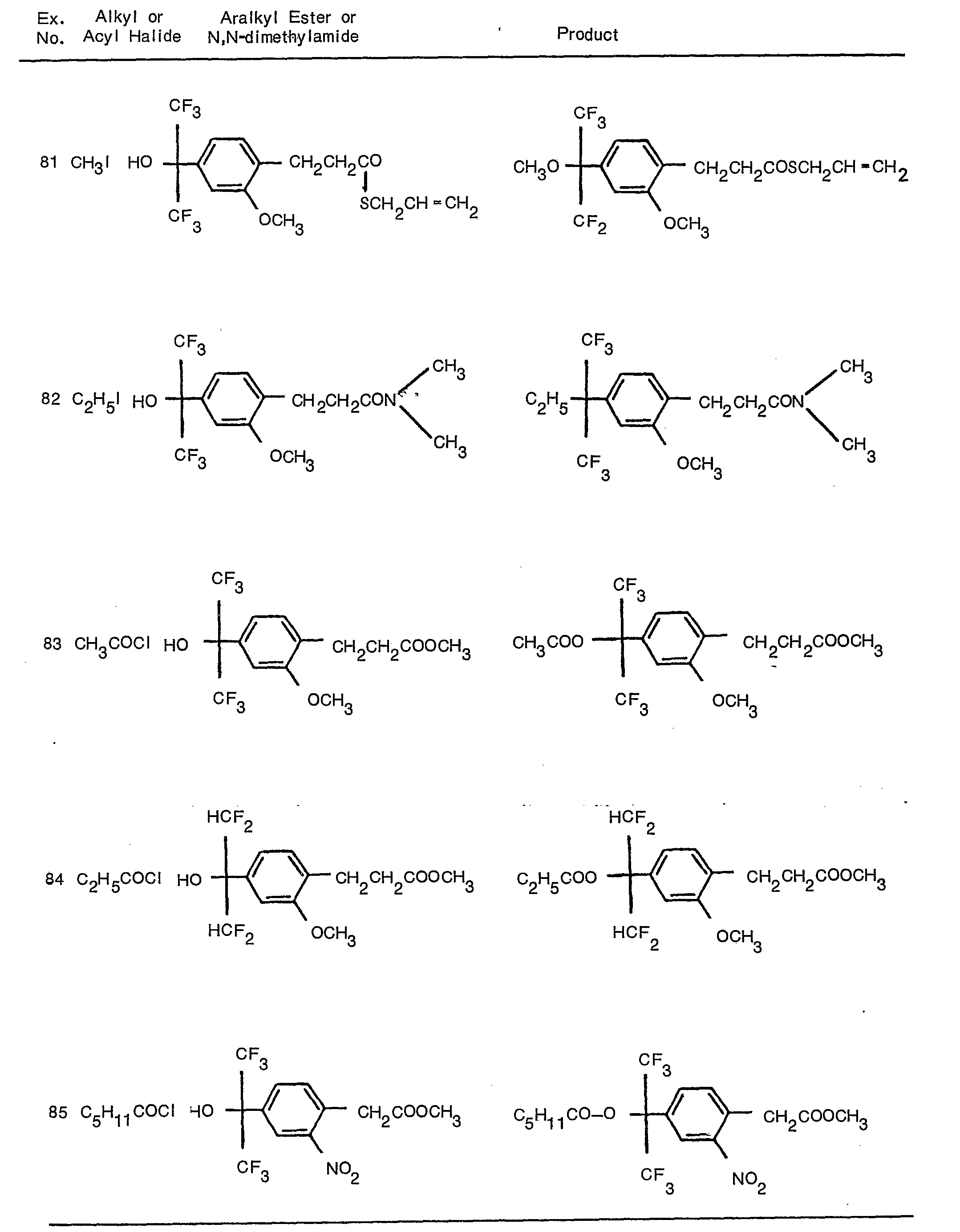

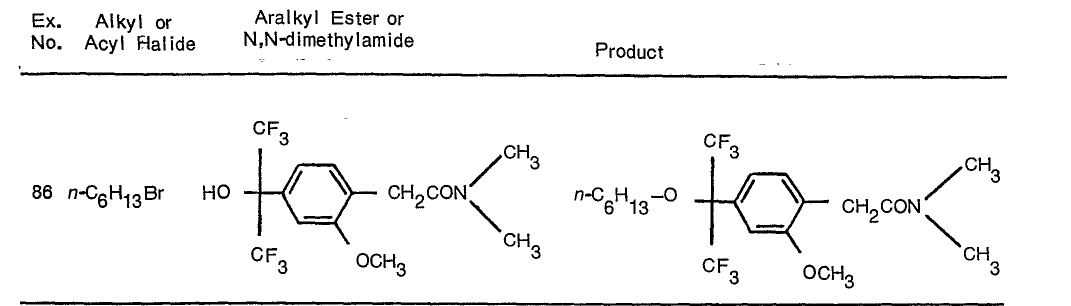

### Dosage

The compounds of this invention can be administered in the treatment of hypertension according to the invention by any means that effects contact of the active ingredient compound with the site of action in the body of a warm-blooded animal. For example, administration can be parenteral, i.e., subcutaneous, intravenous, intramuscular, or intraperitoneal. Alternatively or concurrently, administration can be by the 6ral route.

For the purpose of this disclosure, a warm-blooded animal is a member of the animal kingdom possessed of a homeostatic mechanism and includes mammals and birds.

The dosage administered will be dependent on the age, health and weight of the recipient, the extent of disease, kind of concurrent treatment, if any, frequency of treatment and the nature of the effect desired. Dosages as high as 100 milligrams per kilogram of body weight can be used. Usually, a daily dosage of active ingredient compound will be from about 0.01 to 50 milligrams per kilogram of body weight. Ordinarily, from 0.05 to 40, and preferably 0.1 to 20, milligrams per kilogram per day in one or more applications per day is effective to obtain desired results. For the more potent compounds of the invention, e.g., methyl 2-methoxy-4-[2,2,2-trifluoro-1-hydroxy-1-(trifluoromethyl)ethyl]phenyl- propanoate, the daily dosage ranges are from about 0.01 to 10 mg/kg, preferably 0.05 to 10 mg/kg, and more preferably 0.05 to 5 mg/kg.

Dosage forms (compositions) suitable for internal administration contain from about 0.1 milligrams to about 500 milligrams of active ingredient per unit. In these pharmaceutical compositions the active ingredient will ordinarily be present in an amount of about 0.5-95% by weight based on the total weight of the composition.

The active ingredient can be administered orally in solid dosage forms, such as capsules, tablets, and powders, or in liquid dosage forms, such as elixirs, syrups, and suspensions; it can also be administered parenterally, in sterile liquid dosage forms.

Gelatin capsules can contain the active ingredient and powdered carriers, such as lactose, sucrose, mannitol, starch, cellulose derivatives, magnesium stearate, stearic acid, and the like. Similar diluents can be used to make compressed tablets. Both tablets and capsules can be manufactured as sustained release products to provide for continuous release of medication over a period of hours. Compressed tablets can be sugar coated or film coated to mask any unpleasant taste and protect the tablet from the atmosphere, or enteric coated for selective disintegration in the gastrointestinal tract.

Liquid dosage forms for oral administration can contain coloring and flavoring to increase patient acceptance.

In general, water, a suitable oil, saline, aqueous dextrose (glucose), and related sugar solutions and glycols such as propylene glycol or polyethlene glycols are suitable carriers for parenteral solutions. Solutions for parenteral administration contain preferably a water soluble salt of the active ingredient, suitable stabilizing agents, and if necessary, buffer substances. Antioxidizing agents such as sodium bisulfite, sodium sulfite, or ascorbic acid either alone or combined are suitable stabilizing agents. Also used are citric acid and its salts and sodium EDTA. In addition parenteral solutions can contain preservatives, such as benzalkonium chloride, methyl- or propyl-paraben, and chlorobutanol.

Suitable pharmaceutical carriers are described in Remington's Pharmaceutical Sciences, E. W. Martin, a standard reference text in this field.

Useful pharmaceutical dosage-forms for administration of the compounds of this invention can be illustrated as follows:

### Capsules

A large number of unit capsules can be prepared by filling standard two-piece hard gelatin capsules each with 50 milligrams of powdered active ingredient, 175 milligrams of lactose, 24 milligrams of talc, and 6 milligrams magnesium stearate.

### Capsules

A mixture of active ingredient in soybean oil can be prepared and injected by means of a positive displacement pump into gelatin to form soft gelatin capsules containing 50 milligrams of the active ingredient. The capsules can be washed in petroleum ether and dried.

### Tablets

A large number of tablets can be prepared by conventional procedures so that the dosage unit is 50 milligrams of active ingredient, 6 milligrams of magnesium stearate, 70 milligrams of microcrystalline cellulose, 11 milligrams of cornstarch and 27.5 milligrams of lactose. Appropriate coatings can be applied to increase palatability or delay absorption.

### Injectable

A parenteral composition suitable for administration by injection can be prepared by stirring 1.5% by weight of active ingredient in 10-60% by volume of co-solvents, like propyleneglycol in water. The resultant solution can be sterilized by filtration.

### Suspension

An aqueous suspension can be prepared for oral administration so that each 5 milliliters contain 10 milligrams of finely divided active ingredient, 200 milligrams of sodium carboxymethyl cellulose, 5 milligrams of sodium benzoate, 1.0 grams of sorbitol solution, U.S.P., and 0.025 milliliters of vanillin.

### Injectable

A parenteral composition suitable for administration by injection can be prepared by dissolving 1% by weight of active ingredient in sodium chloride injection U.S.P. XV and adjusting the pH of the solution to between 6 and 7. The solution can be sterilized by filtration.

### Utility

The antihypertensive activity of the compounds of this invention is evidenced by tests conducted in hypertensive rats. In these tests rats are made hypertensive by subcutaneous implantation of pellets of desoxycorticosterone acetate (DOCA) and by giving the rats saline solution to drink essentially according to the method described by Sturtevant (Annals of Internal Medicine, 49, 1281 [1958]). Graded dose levels of each compound are administered orally to groups of 8 hypertensive rats. The compound is prepared in an aqueous polyvinyl alcohol/acacia vehicle and administered at a volume to body weight ratio of 5.0 ml/kg. Sixteen hypertensive rats receiving the aqueous vehicle by the same route serve as controls for each test. At various intervals of time after treatment, usually 90 minutes, the systolic arterial blood pressure of each rat is determined by modification of the microphone-manometer technique (Friedman, M. and Freed, S.C., Proc. Soc. Exp. Biol. and Med., 70, 670 [1949]). That dose of compound which produces a 30 mm mercury (mm Hg) reduction in blood pressure when compared to the mean systolic arterial blood pressure of the control animals is then determined (Effective Dose 30). For example, an ED30 of 0.19 mg/kg orally was obtained with the compound of Example 46. ED 30's of 0.31, 0.45, and 0.23 were obtained with the compounds of Examples 47, 36 and 60; other examples are given in Table 1.

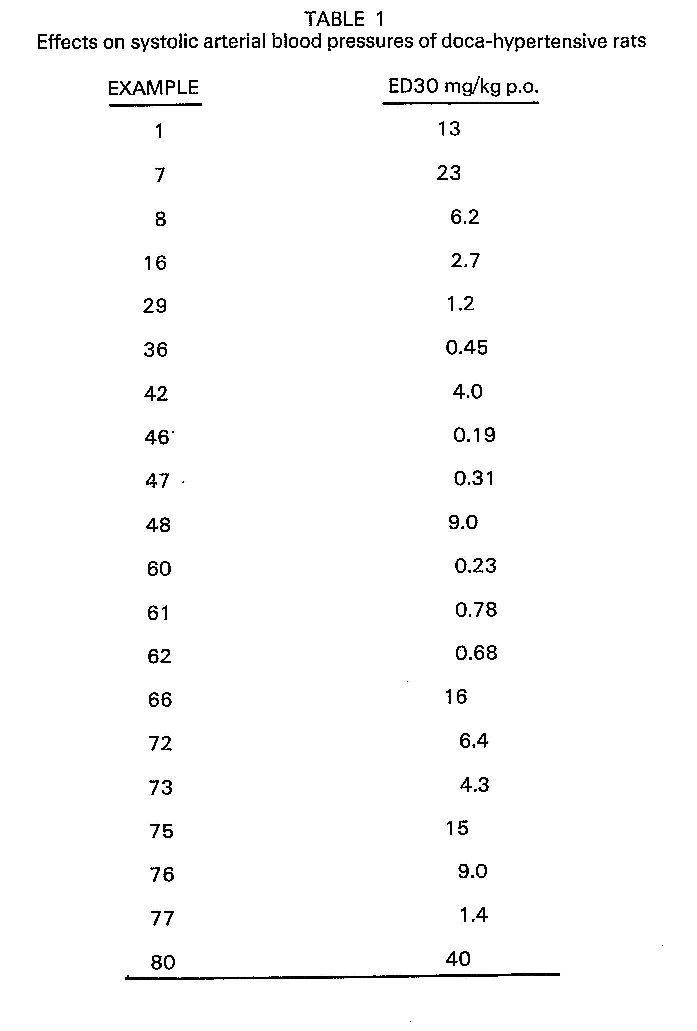

## Claims

1. A compound of the formula 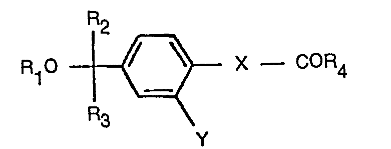 wherein R₁ is hydrogen, alkyl of 1-6 carbon atoms, or acyl of 1-6 carbon atoms;
Y is hydrogen, NO₂, alkoxy of 1-3 carbon atoms, or alkylthio of 1-3 carbon atoms; 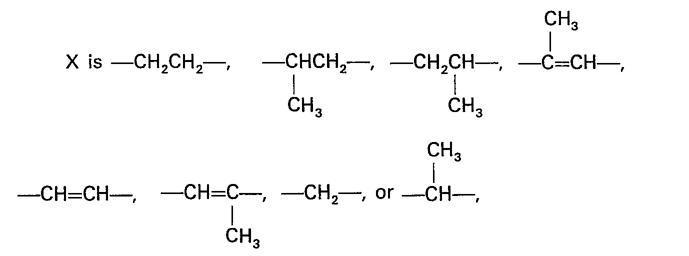 with the proviso that
when Y is H, X is other than - 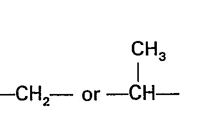 and
when Y is alkylthio, X is other than - 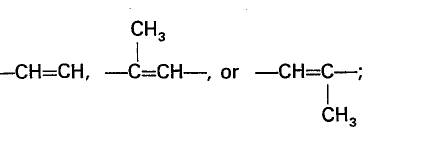
R₄ is N(CH₃)₂, O-Z, S-Z, or CI provided that when Y is H and X is 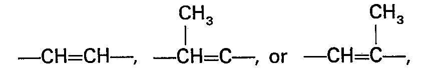 R₄ is other than N(CH₃)₂, wherein
Z is H, alkyl of 1-10 carbon atoms, alkenyl of 3-10 carbon atoms, aryl or aralkyl in which the alkyl group has 1-6 carbon atoms,
with the proviso that when Z is aryl or aralkyl, the aryl group has the formula 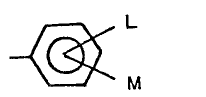 wherein L and M are independently selected from the group consisting of H, F, CI, Br, NO₂, alkyl of 1-4 carbon atoms, alkoxy of 1-4 carbon atoms, CF₃, OCF₃, CN, phenyl, and COOR' where
R' is H, CH₃ or C₂H₅; and
R₂ and R₃ are independently CF₃, CF₂Cl, or CF₂H.

2. A compound of Claim 1 wherein R₄ is ―N(CH₃)₂, O-Z, or S-Z.

3. A compound of Claim 2 wherein R₁ is hydrogen.

4. A compound of Claim 2 wherein Y is OCH₃ or OC₂H₅.

5. A compound of Claim 2 wherein X is ―CH₂CH₂―.

6. A compound of Claim 2 wherein R₄ is OH, OCH₃, OC₂H₅, or N(CH₃)₂.

7. A compound of Claim 2 wherein R₂ and R₃ are CF₃.

8. A compound of Claim 2 wherein R₁ is hydrogen, Y is OCH₃ or OC₂H₅, X is ―CH₂CH₂―, R₄ is OH, OCH₃, OC₂H₅, or N(CH₃)_{z}, and R₂ and R₃ are CF₃.

9. A compound of Claim 8 wherein Y is OCH₃.

10. The compound of Claim 9 which is 3-{2-methoxy-4-[2,2,2-trifluoro-1-hydroxy-1-(trifluoromethyl)ethyl]phenyl}propanoic acid.

11. The compound of Claim 9 which is methyl 2-methoxy-4-[2,2,2-trifluoro-1-hydroxy-1-(trifluoromethyl)ethyl]benzenepropanoate.

12. The compound of Claim 9 which is ethyl 2-methoxy-4-[2,2,2-trifluoro-1-hydroxy-1-(trifluoromethyl)ethyl]benzenepropanoate.

13. The compound of Claim 9 which is 3-{2-methoxy-4-[2,2,2-trifluoro-1-hydroxy-1-(trifluoromethyl)ethyl]phenyl}-N,N-dimethylpropanamide.

14. A pharmaceutical composition consisting essentially of a suitable pharmaceutical carrier and an effective antihypertensive amount of a compound of Claim 2.

15. A pharmaceutical composition consisting essentially of a suitable pharmaceutical carrier and an effective antihypertensive amount of a compound of Claim 3.

16. A pharmaceutical composition consisting essentially of a suitable pharmaceutical carrier and an effective antihypertensive amount of a compound of Claim 4.

17. A pharmaceutical composition consisting essentially of a suitable pharmaceutical carrier and an effective antihypertensive amount of a compound of Claim 5.

18. A pharmaceutical composition consisting essentially of a suitable pharmaceutical carrier and an effective antihypertensive amount of a compound of Claim 6.

19. A pharmaceutical composition consisting essentially of a suitable pharmaceutical carrier and an effective antihypertensive amount of a compound of Claim 7.

20. A pharmaceutical composition consisting essentially of a suitable pharmaceutical carrier and an effective antihypertensive amount of a compound of Claim 8.

21. A pharmaceutical composition consisting essentially of a suitable pharmaceutical carrier and an effective antihypertensive amount of a compound of Claim 9.

22. A pharmaceutical composition consisting essentially of a suitable pharmaceutical carrier and an effective antihypertensive amount of the compound of Claim 10.

23. A pharmaceutical composition consisting essentially of a suitable pharmaceutical carrier and an effective antihypertensive amount of the compound of Claim 11.

24. A pharmaceutical composition consisting essentially of a suitable pharmaceutical carrier and an effective antihypertensive amount of the compound of Claim 12.

25. A pharmaceutical composition consisting essentially of a suitable pharmaceutical carrier and an effective antihypertensive amount of the compound of Claim 13.

## Revendications

1. Un composé de la formule 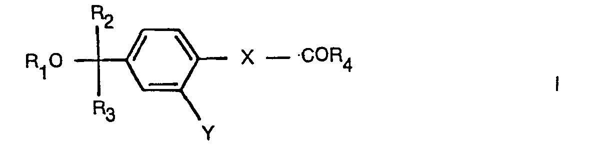 dans laquelle
R, est l'hydrogène, un groupe alcoyle de 1 à 6 atomes de carbone ou un groupe acyle de 1 à 6 atomes de carbone;
Y est l'hydrogène, NO₂, un groupe alcoxy de 1 à 3 atomes de carbone ou un groupe alcoylthio de 1 à 3 atomes de carbone; 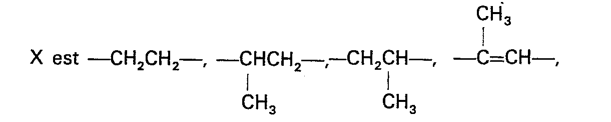 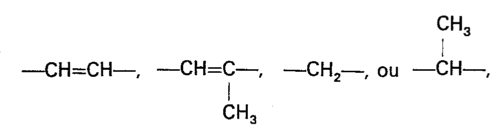 avec les conditions que, quand Y est H, alors X est autre que 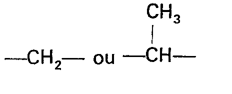 et, quand Y est un groupe alcoylthio, alors X est autre que 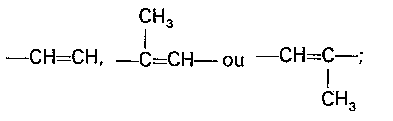
R₄ est N(CH₃)₂, O―Z, S-Z ou CI, avec la condition que, quand Y est H et X est - 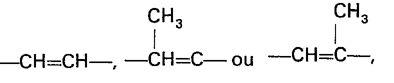 alors R₄ est autre que N(CH₃)₁, où
Z est H, un groupe alcoyle de 1 à 10 atomes de carbone, alcényle de 3 à 10 atomes de carbone, aryle ou aralcoyle où le groupe alcoyle a de 1 à 6 atomes de carbone, avec la condition que, quand Z est un groupe aryle ou aralcoyle, alors le groupe aryle a la formule 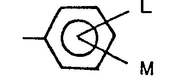 dans laquelle L et M sont choisis indépendamment parmi H, F, CI, Br, NO₂, un groupe alcoyle de 1 à 4 atomes de carbone, alcoxy de 1 à 4 atomes de carbone, CF₃, OCF₃, CN, phényle et COOR', où R' est H, CH₃ ou C₂H₅; et
R₂ et R₃ sont indépendamment CF₃, CF₂Cl ou CF₂H.

2. Un composé selon la revendication 1, dans lequel R₄ est ―N(CH₃)₂, O―Z ou S-Z.

3. Un composé selon la revendication 2, dans lequel Rᵢ est l'hydrogène.

4. Un composé selon la revendication 2, dans lequel Y est OCH₃ ou OC₂H₅.

5. Un composé selon la revendication 2, dans lequel X est ―CH₂CH₂―.

6. Un composé selon la revendication 2, dans lequel R₄ est OH, OCH₃, OC₂H₅ ou N(CH₃)₂.

7. Un composé selon la revendication 2, dans lequel R_{z} et R₃ sont CF₃.

8. Un composé selon la revendication 2, dans lequel R₁ est l'hydrogène, Y est OCH₃ ou OC₂H₅, X est ―CH₂CH₂―, R₄ est OH, OCH₃, OC₂H₅ ou N(CH₃)₂ et R₂ et R₃ sont CF₃.

9. Un composé selon la revendication 8, dans lequel Y est OCH₃.

10. Le composé selon la revendication 9 qui est l'acide 3-{2-méthoxy-4-[2,2,2-trifluoro-1-hydroxy-1-(trifluorométhyl)éthyl]phényl}propanoïque.

11. Le composé selon la revendication 9 qui est le 2-méthoxy-4-[2,2,2-trifluoro-1-hydroxy-1-trifluorométhyl)éthyl]benzènepropanoate de méthyle.

12. Le composé selon la revendication 9 qui est le 2-méthoxy-4-[2,2,2-trifluoro-1-hydroxy-1-(tri- fluorométhyl)éthyllbenzènepropanoate d'éthyle.

13. Le composé selon la revendication 9 qui est le 3-{2-méthoxy-[2,2,2-trifluoro-1-hydroxy-1-(trifluorométhyl)éthyl]phényl}-N,N-diméthylpropanamide.

14. Une composition pharmaceutique constituée essentiellement d'un véhicule pharmaceutique et d'une quantité hypertensive efficace d'un composé selon la revendication 2.

15. Une composition pharmaceutique constituée essentiellement d'un véhicule pharmaceutique et d'une quantité hypertensive efficace d'un composé selon la revendication 3.

16. Une composition pharmaceutique constituée essentiellement d'un véhicule pharmaceutique et d'une quantité hypertensive efficace d'un composé selon la revendication 4.

17. Une composition pharmaceutique constituée essentiellement d'un véhicule pharmaceutique et d'une quantité hypertensive efficace d'un composé selon la revendication 5.

18. Une composition pharmaceutique constituée essentiellement d'un véhicule pharmace.utique et d'une quantité hypertensive efficace d'un composé selon la revendication 6.

19. Une composition pharmaceutique constituée essentiellement d'un véhicule pharmaceutique et d'une quantité hypertensive efficace d'un composé selon la revendication 7.

20. Une composition pharmaceutique constituée essentiellement d'une véhicule pharmaceutique et d'une quantité hypertensive efficace d'un composé selon la revendication 8.

21. Une composition pharmaceutique constituée essentiellement d'un véhicule pharmaceutique et d'une quantité hypertensive efficace d'un composé selon la revendication 9.

22. Une composition pharmaceutique constituée essentiellement d'un véhicule pharmaceutique et d'une quantité hypertensive efficace d'une composé selon la revendication 10.

23. Une composition pharmaceutique constituée essentiellement d'un véhicule pharmaceutique et d'une quantité hypertensive efficace d'un composé selon la revendication 11.

24. Une composition pharmaceutique constituée essentiellement d'un véhicule pharmaceutique et d'une quantité hypertensive efficace d'un composé selon la revendication 12.

25. Une composition pharmaceutique constituée essentiellement d'un véhicule pharmaceutique et d'une quantité hypertensive efficace d'un composé selon la revendication 13.

## Patentansprüche

1. Verbindung der Formel 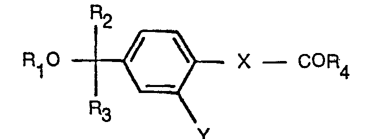 in der
R₁ Wasserstoff, Alkyl mit 1 bis 6 Kohlenstoff-Atomen oder Acyl mit 1 bis 6 Kohlenstoff-Atomen ist,
Y Wasserstoff, NO₂, Alkoxy mit 1 bis 3 Kohlenstoff-Atomen oder Alkylthio mit 1 bis 3 Kohlenstoff-Atomen ist, 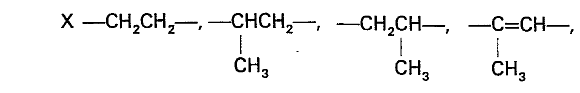 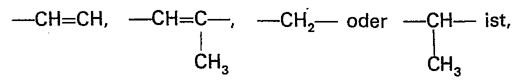 mit der Maßgabe, daß, wenn Y H ist 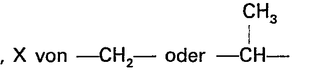 verschieden ist, und wenn Y Alkylthio ist, 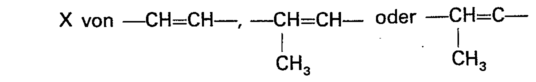 verschieden ist,
R₄ N(CH₃)₂, O-Z, S-Z oder CI ist, unter der Voraussetzung, daß, wenn Y H und X 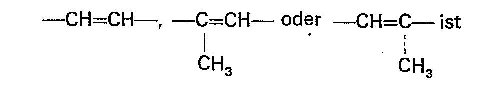
R₄ von N(CH₃)₂ verschieden ist, wobei
Z H, Alkyl mit 1 bis 10 Kohlenstoff-Atomen, Alkenyl mit 3 bis 10 Kohlenstoff-Atomen, Aryl oder Aralkyl, in dem die Alkyl-Gruppe 1 bis 6 Kohlenstoff-Atome besitzt, ist, mit der Maßgabe, daß, wenn Z Aryl oder Aralkyl ist, die Aryl-Gruppe die Formel 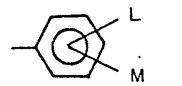 besitzt, in der L und M unabhängig voneinander ausgewählt sind aus der aus H, F, Cl, Br, NO₂, Alkyl mit 1 bist 4 Kohlenstoff-Atomen, Alkoxy mit 1 bis 4 Kohlenstoff-Atomen, CF₃, OCF₃, CN, Phenyl und COOR', worin R' H, CH₃ oder C₂H₅ ist, bestehenden Gruppe und R₂ und R₃ unabhängig voneinander CF₃, CF₂Cl oder CF₂H sind.

2. Verbindung nach Anspruch 1, dadurch gekennzeichnet, daß R₄ ―N(CH₃)₂, 0-Z oder S-Z ist.

3. Verbindung nach Anspruch 2, dadurch gekennzeichnet, daß R, Wasserstoff ist.

4. Verbindung nach Anspruch 2, dadurch gekennzeichnet, daß Y OCH₃ oder OC₂H₅ ist.

5. Verbindung nach Anspruch 2, dadurch gekennzeichnet, daß X ―CH₂CH₂― ist.

6. Verbindung nach Anspruch 2, dadurch gekennzeichnet, daß R₄ OH, OCH₃, OC₂H₅ oder N(CH₃)₂ ist.

7. Verbindung nach Anspruch 2, dadurch gekennzeichnet, daß R₂ und R₃ CF₃ sind.

8. Verbindung nach Anspruch 2, dadurch gekennzeichnet, daß R, Wasserstoff ist, Y OCH₃ oder 0C₂H₅ ist, X ―CH₂CH₂― ist, R₄ OH, OCH₃, OC_{z}Hₛ oder N(CH₃)₂ ist und R₂ und R₃ CF₃ sind.

9. Verbindung nach Anspruch 8, dadurch gekennzeichnet, daß Y OCH₃ ist.

10. Verbindung 3-{-2-Methoxy-4-[2,2,2-trifluoro-1-hydroxy-1-(trifluoromethyl)ethyl]phenyl}- propansäure nach Anspruch 9.

11. Verbindung Methyl-2-methoxy-4-[2,2,2-trifluoro-1-hydroxy-1-(trifluoromethyl)ethyl]benzol- propanoat nach Anspruch 9.

12. Verbindung Ethyl-2-methoxy-4-[2,2,2-trifluoro-1-hydroxy-1-(trifluoromethyl)ethyl]benzol- propanoat nach Anspruch 9.

13. Verbindung 3-{-2-Methoxy-4-[2,2,2-trifluoro-1-hydroxy-1-(trifluoromethyl)ethyl]phenyl}-N,N-dimethylpropanamid nach Anspruch 9.

14. Pharmazeutische Zusammensetzung bestehend im wesentlichen aus einem geeigneten pharmazeutischen Träger und einer antihypertonisch wirksamen Menge einer Verbindung nach Anspruch 2.

15. Pharmazeutische Zusammensetzung bestehend im wesentlichen aus einem geeigneten pharmazeutischen Träger und einer antihypertonisch wirksamen Menge einer Verbindung nach Anspruch 3.

16. Pharmazeutische Zusammensetzung bestehend im wesentlichen aus einem geeigneten pharmazeutischen Träger und einer antihypertonisch wirksamen Menge einer Verbindung nach Anspruch 4.

17. Pharmazeutische Zusammensetzung bestehend im wesentlichen aus einem geeigneten pharmazeutischen Träger und einer antihypertonisch wirksamen Menge einer Verbindung nach Anspruch 5.

18. Pharmazeutische Zusammensetzung bestehend im wesentlichen aus einem geeigneten pharmazeutischen Träger und einer antihypertonisch wirksamen Menge einer Verbindung nach Anspruch 6.

19. Pharmazeutische Zusammensetzung bestehend im wesentlichen aus einem geeigneten pharmazeutischen Träger und einer antihypertonisch wirksamen Menge einer Verbindung nach Anspruch 7.

20. Pharmazeutische Zusammensetzung bestehend im wesentlichen aus einem geeigneten pharmazeutischen Träger und einer antihypertonisch wirksamen Menge einer Verbindung nach Anspruch 8.

21. Pharmazeutische Zusammensetzung bestehend im wesentlichen aus einem geeigneten pharmazeutischen Träger und einer antihypertonisch wirksamen Menge einer Verbindung nach Anspruch 9.

22. Pharmazeutische Zusammensetzung bestehend im wesentlichen aus einem geeigneten pharmazeutischen Träger und einer antihypertonisch wirksamen Menge einer Verbindung nach Anspruch 10.

23. Pharmazeutische Zusammensetzung bestehend im wesentlichen aus einem geeigneten pharmazeutischen Träger und einer antihypertonisch wirksamen Menge einer Verbindung nach Anspruch 11.

24. Pharmazeutische Zusammensetzung bestehend im wesentlichen aus einem geeigneten pharmazeutischen Träger und einer antihypertonisch wirksamen Menge einer Verbindung nach Anspruch 12.

25. Pharmazeutische Zusammensetzung bestehend im wesentlichen aus einem geeigneten pharmazeutischen Träger und einer antihypertonisch wirksamen Menge einer Verbindung nach Anspruch 13.
